# EUROPÄISCHE PATENTSCHRIFT

(11) **EP 4 665 791 B1**
(45) Veröffentlichungstag und Bekanntmachung des Hinweises auf die Patenterteilung: **14.10.2026**
(21) Anmeldenummer: 24704481.1
(22) Anmeldetag: 13.02.2024
(51) Int. Cl.: C08J 11/24, C07C 263/04, C07C 263/20

(54) **VERFAHREN ZUR WIEDERGEWINNUNG VON ROHSTOFFEN AUS POLYESTERPOLYURETHAN- ELASTOMER-HALTIGEN POLYMERZUSAMMENSETZUNGEN**
PROCESS FOR RECOVERING RAW MATERIALS FROM POLYESTER-POLYURETHANE ELASTOMER-CONTAINING POLYMER COMPOSITIONS
PROCÉDÉ DE RÉCUPÉRATION DE MATIÈRES PREMIÈRES À PARTIR DE COMPOSITIONS POLYMÈRES CONTENANT DES ÉLASTOMÈRES DE POLYESTER-POLYURÉTHANE

(30) Priorität: 14.02.2023 EP 23156546; 29.11.2023 EP 23212857
(43) Veröffentlichungstag der Anmeldung: 24.12.2025
(73) Patentinhaber: Covestro Deutschland AG, 51373 Leverkusen (DE)
(72) Erfinder: KLOECKNER, Wolf, 51065 Köln (DE); RICHTER, Frank, 51373 Leverkusen (DE); RADATZ, Heiko, 03205 Calau (DE); KIECHERER, Johannes, 50969 Köln (DE); LEVEN, Matthias, 51069 Köln (DE); WALKER, Karolina, 50739 Köln (DE); SCHUMACHER, Hannah, 50735 Köln (DE); ALTUNTEPE, Emrah, 40764 Langenfeld (DE); FRANCISCO CASAL, Maria, 51375 Leverkusen (DE); REISKY, Lukas, 50996 Köln (DE); MAJIDI SALEHI, Shabnam, 40591 Düsseldorf (DE); MACHAT, Martin, 50735 Köln (DE); LARCHER, Christoph, 40476 Düsseldorf (DE); RENGSHAUSEN, Simon, 51379 Leverkusen (DE); EL SAYED, Sami, 52066 Aachen (DE); SCHROERS, Julian, 52062 Aachen (DE); LEITNER, Walter, 52074 Aachen (DE); KLANKERMAYER, Jürgen, 52074 Aachen (DE)
(74) Vertreter: Levpat
(86) Internationale Anmeldenummer: PCT/EP2024/053613
(87) Internationale Veröffentlichungsnummer: WO 2024/170561

(56) Entgegenhaltungen:
- WO-A1-2023/285545
- DE-A1- 4 431 961

## Beschreibung

Die vorliegende Erfindung betrifft ein Verfahren zur Wiedergewinnung von Rohstoffen aus Polyester-Polyurethan-Elastomer-haltigen Polymerzusammensetzungen, umfassend die Schritte **(A)** Bereitstellen einer Polymerzusammensetzung enthaltend ein auf **(i)** einer Polyesterkomponente, **(ii)** einer Isocyanatkomponente und optional **(iii)** einem Kettenverlängerer und/oder Vernetzer mit Hydroxy- und/oder Aminfunktionalität basierendes Polyester-Polyurethan-Elastomer, wobei die Polymerzusammensetzung ein erstes Polyester-Polyurethan-Elastomer enthält, dessen Isocyanatkomponente Naphthylen-1,5-diisocyanat umfasst; **(B)** Umsetzen der Polymerzusammensetzung mit einem linearen primären Mono-Alkohol mit 1 bis 4 Kohlenstoffatomen unter Erhalt eines Produktgemisches umfassend eine flüssige Alkoholphase und eine feste Carbamatphase; und **(C)** Isolieren des Carbamats aus dem Produktgemisch.

Polyurethane finden vielfältige Anwendungen in der Industrie und im Alltag. Üblicherweise wird bei den Polyurethanen zwischen Polyurethanschäumen und sog. "CASE"-Produkten unterschieden, wobei "CASE" ein Sammelbegriff für *Polyurethan-Beschichtungen* (z. B. Lacke), *-Klebstoffe, -Dichtstoffe* und *-Elastomere* ist. Die Polyurethanschäume werden üblicherweise in Hartschäume und Weichschäume unterteilt. All diesen Produkten ist trotz ihrer Verschiedenheit die Polyurethangrundstruktur gemeinsam, die durch die Polyadditionsreaktion eines mehrwertigen Isocyanats und eines Polyols entsteht und sich beispielsweise für ein Polyurethan, das auf einem Diisocyanat **O=C=N-R-N=C=O** und einem Diol H-O-R'-O-H basiert (wobei R und R' organische Reste bezeichnen) als

**^{~~~}**[O-R'-O-**(O=C)-**H**N-R-N**H**-(C=O)]^{~~~}**

darstellen lässt.

Viele Polyurethane enthalten neben der Polyurethangrundstruktur weitere Struktureinheiten. Hier seien insbesondere Harnstoff-, Isocyanurat-, Allophanat- und Biuretstruktureinheiten genannt.

Gerade wegen des großen wirtschaftlichen Erfolges der Polyurethane fallen auch große Mengen an Polyurethanabfall an, der einer sinnvollen Verwendung zugeführt werden muss. Die technisch am einfachsten umzusetzende Art der Wiederverwendung ist die Verbrennung unter Nutzung der freiwerdenden Verbrennungswärme für andere Prozesse, beispielweise industrielle Herstellprozesse. Auf diese Weise ist jedoch eine Schließung der Rohstoffkreisläufe nicht möglich. Eine andere Art der Wiederverwendung ist das sog. "*physikalische* Recycling", bei welchem Polyurethanabfälle mechanisch zerkleinert und in der Herstellung neuer Produkte eingesetzt werden. Dieser Art des Recyclings sind naturgemäß Grenzen gesetzt, weshalb es nicht an Versuchen gefehlt hat, die der Polyurethanproduktion *zugrunde liegenden Rohstoffe* durch Rückspaltung der Urethanbindungen (und der gegebenenfalls darüber hinaus vorhandenen Bindungsstrukturen wie Isocyanurat-, Harnstoff-, Allophanat- oder Biuret,-bindungen) wiederzugewinnen (sog. "*chemisches* Recycling"). Die zurückzugewinnenden Rohstoffe umfassen dabei in erster Linie Polyole (im obigen Beispiel also H-O-R'-O-H) oder deren Abbauprodukte (z. B. die einem Polyesterpolyol zugrunde liegenden Monomere). Daneben können durch hydrolytische Spaltung der Urethanbindung auch Amine gewonnen werden (im obigen Beispiel also H₂N-R-NH₂), die sich nach Aufarbeitung zu Isocyanaten (im obigen Beispiel zu O=C=N-R-N=C=O) phosgenieren lassen.

Verschiedene Ansätze des chemischen Recyclings wurden in der Vergangenheit entwickelt. Die drei wichtigsten werden im Folgenden kurz zusammengefasst:
1. **Hydrolyse** von Urethanen durch Umsetzung mit Wasser unter Gewinnung von Aminen und Polyolen unter Bildung von Kohlenstoffdioxid.
**2. Glykolyse** von Urethanen durch Umsetzung mit Alkoholen, wobei die Polyole, die in den Urethangruppen eingebaut sind, durch den eingesetzten Alkohol ersetzt und auf diese Weise freigesetzt werden. Dieser Vorgang wird in der Literatur meist als *Umesterung* bezeichnet (genauer: *Umurethanisierung).* Diese Art des chemischen Recyclings wird unabhängig von der genauen Art des eingesetzten Alkohols in der Literatur üblicherweise als *Glykolyse* bezeichnet, obwohl dieser Begriff eigentlich nur für Glykol oder Glykolderivate zutreffend ist. (Im Zusammenhang mit der vorliegenden Erfindung wird daher allgemein von *Alkoholyse* gesprochen.) An eine Glykolyse kann sich eine Hydrolyse anschließen. Wird die Hydrolyse mit dem unmittelbaren Verfahrensprodukt der Glykolyse (also ohne vorherige Trennung von Polyolen und Carbamaten) durchgeführt, spricht man von einer
3. **Hydroglykolyse** von Urethanbindungen durch Umsetzung mit Alkoholen *und* Wasser. Es ist natürlich ebenfalls möglich, Alkohol und Wasser von Anfang an zuzugeben, wobei die oben beschriebenen Vorgänge der Glykolyse und Hydrolyse parallel ablaufen.

Der in Polymer Degradation and Stability 2004, 86, 147 - 151 erschienene Artikel Methanolysis investigation of commercially available polyurethane foam von N. Asahi et al. beschreibt die Methanolyse von Polyurethanen im Temperaturbereich von 160 bis 300 °C bei Drücken von bis zu 15 MPa, wobei das Methanol teilweise in überkritischem Zustand vorlag. Es war angestrebt, die Methanolyse eines kommerziell erhältlichen Polyurethanschaums ohne Einsatz eines Katalysators zu verwirklichen. Untersucht wurden die Chemolyse des Modell-Polyurethans 1, 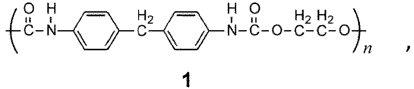 und die des kommerziell erhältlichen Polyurethanschaums "Sofran^{®}" bei Massenverhältnissen von Methanol zu Polyurethan von 5 : 1 bzw. 16 : 1. Der Artikel kommt zu der Schlussfolgerung, dass die Zersetzung des Polyurethans zu Methylcarbamat in hohen Anteilen bei Temperaturen oberhalb von 200 °C gelingt und dass bei niedrigeren Temperaturen der Einsatz von Katalysatoren erforderlich sei.

WO 2023/285545 A1 beschreibt ein Verfahren zum Recycling eines Polyurethans, bei dem das Polyurethan mit einem ersten Alkohol umgesetzt und das dabei oder in einer weiteren Umurethanisierung mit einem zweiten Alkohol gebildete niedermolekulare Carbamat thermisch in den zugrundeliegenden Alkohol und in das zugrundeliegende Isocyanat gespalten wird. Besonders bevorzugte erste und/oder zweite Alkohole sind Propanol, Pentanol, Isopropylalkohol, Butanol, Hexanol, Nonanol, Octanol, Glycerin, Ethylenglylol, Diethylenglykol und Triethylenglykol. In den Beispielen wird ein Polyurethanweichschaum einmal mit Octanol (Massenverhältnis Schaum zu Alkohol 1 : 2) und einmal mit *iso*Propanol (Massenverhältnis Schaum zu Alkohol 1 : 5) umgesetzt.

EP 3 590 999 B1 beschreibt ein Verfahren zum Abbau von Kunststoffen mit Methanol oder Ethanol in Gegenwart von Methanolat als Katalysator. In den Beispielen wird der Abbau von Polyurethanweichschaum beschrieben.

Eine Zusammenfassung der bekannten Verfahren des Polyurethanrecyclings bietet der Übersichtsartikel von Simón, Borreguero, Lucas und Rodriguez in Waste Management 2018, 76, 147 - 171 [1].

Der Fokus der Bemühungen zur Wiederverwertung von Polyurethanen in der Literatur liegt auf Polyurethanschäumen. Eine wichtige Klasse von Polyurethanen sind jedoch auch die Elastomere, die zu den oben genannten "CASE"-Produkten gehören. Hier wiederum stellt die Gruppe der Polyester-Polyurethan-Elastomere (auch kurz Polyester-Urethan-Elastomere oder Polyester-Urethan-Kautschuke genannt) eine wichtige Verbindungsklasse dar, die sich durch elastische Eigenschaften ähnlich dem Naturgummi bei besserer chemischer und mechanischer Beständigkeit auszeichnen. Für besonders hochwertige Produkte wird Naphthylen-1,5-diisocyanat (fortan: 1,5-NDI) als Isocyanatkomponente benutzt. Entsprechende Elastomere sind unter dem Markennamen Vulkollan^{®} bekannt. Massives Vulkollan^{®} wird durch chemische Reaktionen von 1,5-NDI, Polyesterpolyolen und Glykolen hergestellt. Es wird für die Produktion von Rädern und Rollen für höchste dynamische Belastungen sowie technische Teile und Halbzeuge verwendet. Zelliges Vulkollan^{®} wird aus 1,5-NDI, Polyesterpolyolen und Wasser hergestellt. Es vereint hohe Volumenkompressibilität mit minimaler Querdehnung. Zelliges Vulkollan^{®} wird für die Herstellung hochwertiger und leistungsstarker Dämpfungselemente wie Puffer, Federn und NVH (Noise, Vibration, Harshness)-Komponenten eingesetzt. Massive und zellige NDI-basierte Polyurethan-Gießelastomere können auch aus NDI-Präpolymeren hergestellt werden.

DE 44 31 961 A1 beschreibt ein Verfahren zur Gewinnung von Naphthylen-1,5-diamin (1,5-NDA) durch Hydrolyse von auf Basis von 1,5-NDI, gegebenenfalls auch 1,5-NDA, hergestellten Polyurethanen und/oder Polyurethanpolyharnstoffen. Die Hydrolyse erfolgt mit Wasser bei Temperaturen von 170 bis 250 °C, gegebenenfalls bei Drücken von 6 bis 100 bar, in Gegenwart von mindestens einer der Urethangruppen äquivalenten Menge eines oder mehrerer Metallhydroxide und/oder Metalloxide im pH-Bereich von 8,5 bis 14. Das Verfahren zeichnet sich durch eine besonders hohe Reinheit des gewonnenen 1,5-NDA aus. Die Hydrolyse eines in einem vorgelagerten Glykolyseschritt (mit Dipropylenglykol) gewonnenen niedermolekularen NDI-Carbamats wird als nachteilig geschildert. Das in DE 44 31 961 A1 beschriebene Hydrolyseverfahren erfordert den Einsatz großer Mengen starker Basen, was teuer ist und den Aufwand bei Aufarbeitung und Abwasseraufbereitung erhöht. Im Verfahren gemäß DE 44 31 961 A1 ist infolge der Durchführung der Chemolyse als direkte Hydrolyse die Möglichkeit, aus den eingesetzten Polyurethanen und/oder Polyurethanpolyharnstoffen ein niedermolekulares Carbamat zu gewinnen und dieses *anderen Verarbeitungsstufen als einer chemischen Hydrolyse* zuzuführen, von vorne herein ausgeschlossen.

Ein praktikables und flexibles Recycling-Verfahren für Polyester-Polyurethan-Elastomere auf 1,5-NDI-Basis ist daher äußerst wünschenswert. Der Stand der Technik bietet hierzu noch keine umfassende Lösung.

Es bestand also ein Bedarf an weiteren Verbesserungen auf dem Gebiet des Polyurethanrecyclings. Insbesondere wäre es wünschenswert, ein Verfahren zur Wiederverwertung von Polyester-Polyurethan-Elastomeren auf 1,5-NDI-Basis zur Verfügung zu haben, das es gestattet, wichtige Rohstoffe, insbesondere 1,5-NDI (bzw. dessen Vorläufer, Naphthylen-1,5-diamin, 1,5-NDA), idealerweise auch Bausteine der Polyesterkomponente und/oder Kettenverlängerer und Vernetzer, wiederzugewinnen.

Diesem Bedarf Rechnung tragend ist ein Gegenstand der vorliegenden Erfindung ein **Verfahren zur Wiedergewinnung von Rohstoffen** (mindestens eines Carbamats) aus Polyester-Polyurethan-Elastomer-haltigen Polymerzusammensetzungen, umfassend die Schritte:
(A) Bereitstellen einer Polymerzusammensetzung enthaltend (mindestens) ein auf (i) einer Polyesterkomponente, (ii) einer Isocyanatkomponente und optional (iii) einem Kettenverlängerer und/oder Vernetzer mit Hydroxy- und/oder Aminfunktionalität, insbesondere einem Alkohol oder Aminoalkohol mit zwei oder mehr, bevorzugt zwei bis drei, Hydroxygruppen, insbesondere mit 1 bis 10 Kohlenstoffatomen, basierendes
   Polyester-Polyurethan-Elastomer,
   wobei die Polymerzusammensetzung ein erstes Polyester-Polyurethan-Elastomer enthält, dessen Isocyanatkomponente Naphthylen-1,5-diisocyanat umfasst (und bevorzugt neben Naphthylen-1,5-diisocyanat allenfalls noch weitere Isomere des Naphthylendiisocyanats, wie insbesondere Naphthylen-2,6-diisocyanat und Naphthylen-1,8-diisocyanat, aber keine von Naphthylendiisocyanat (NDI) verschiedenen Isocyanate umfasst, wobei das erste Polyester-Polyurethan-Elastomer auch ein Gemisch verschiedener Polyester-Polyurethan-Elastomere mit einer Isocyanatkomponente umfassend Naphthylen-1,5-diisocyanat sein kann);
(B) Umsetzen der Polymerzusammensetzung mit einem (überstöchiometrisch eingesetzten) linearen primären Mono-Alkohol mit 1 bis 4 Kohlenstoffatomen (fortan auch als *Chemolysealkohol* bezeichnet) unter Erhalt eines Produktgemisches umfassend
   eine flüssige Alkoholphase (enthaltend (i) nicht umgesetzten linearen primären Mono-Alkohol, (ii) Produkte der Alkoholyse der Urethanbindungen und Esterbindungen, und (iii) gegebenenfalls Diol);
      und
   eine feste Carbamatphase (enthaltend ein (Mono- und/oder Bis-, insbesondere Bis-)Carbamat des Naphthylen-1,5-diisocyanats und des linearen primären Mono-Alkohols);
   und
(C) Isolieren des Carbamats aus dem Produktgemisch.

Vollkommen überraschend wurde gefunden, dass die Überführung des 1,5-NDI-Bausteins in ein niedermolekulares Carbamat eines linearen primären C₁-C₄-Alkanols auf einfache Weise gelingt, wobei dessen Isolierung in fester Form möglich ist und die Trennung der Bestandteile des Chemolyseproduktes voneinander erheblich vereinfacht wird. Auf diese Weise wird ebenfalls die Rückgewinnung monomerer Bausteine der Polyesterkomponente und/oder von Kettenverlängerern bzw. Vernetzern möglich.

In der Terminologie der vorliegenden Erfindung umfasst der Begriff "*Polymerzusammensetzung enthaltend (mindestens) ein* ... *Polyester-Polyurethan-Elastomer"* die Gesamtheit der in Schritt (B) umzusetzenden Polyester-Polyurethan-Elastomere, worunter sich mindestens ein 1,5-NDI-basiertes Polyester-Polyurethan-Elastomer, im Rahmen der vorliegenden Erfindung als *erstes Polyester-Polyurethan-Elastomer* bezeichnet, befindet. Im einfachsten Fall gibt es keine weiteren Polyester-Polyurethan-Elastomere, dann ist die dem Schritt (B) zuzuführende Polymerzusammensetzung (gegebenenfalls abgesehen von mitgeschleppten Verunreinigungen, die beispielsweise aus dem ursprünglichen Gebrauch des Polyester-Polyurethan-Elastomers stammen können) identisch mit dem 1,5-NDI-basierten Polyester-Polyurethan-Elastomer. Es können auch mehrere verschiedene 1,5-NDI-basierte Polyester-Polyurethan-Elastomere vorhanden sein, die sich etwa in der Art der Polyesterkomponente unterscheiden. In einem solchen Fall gilt die Gesamtheit aller 1,5-NDI-basierten Polyester-Polyurethan-Elastomere als *erstes Polyester-Polyurethan-Elastomer* im Sinne der vorliegenden Erfindung. Wie oben bereits erwähnt, kann ein 1,5-NDI-basiertes Polyester-Polyurethan-Elastomer auch (geringe) Anteile an anderen Isomeren des NDI enthalten; dies verlässt den Rahmen der vorliegenden Erfindung nicht. Bevorzugt betragen die Massenanteile an NDI-Isomeren, die von 1,5-NDI verschieden sind, 0,0 % bis 2,0 %, besonders bevorzugt 0,0 % bis 1,0 %, ganz besonders bevorzugt 0,0 % bis 0,8 % und außerordentlich ganz besonders bevorzugt 0,0 % bis 0,5 %, bezogen auf die Gesamtmasse allen vorhandenen Naphthylendiisocyanats.

Wie weiter unten noch erläutert wird, können in bestimmten Ausführungsformen weitere Polyester-Polyurethan-Elastomere, die auf anderen Isocyanaten als 1,5-NDI basieren, in der Polymerzusammensetzung vorhanden sein. Mit solchen anderen Isocyanaten sind Isocyanate mit einem anderen Kohlenstoffgrundgerüst gemeint und nicht etwa NDI-Komponenten, die sich nur durch eine geringfügig verschiedene Isomerenverteilung unterscheiden. Diese anderen Polyester-Polyurethan-Elastomere werden im Folgenden in ihrer Gesamtheit als *zweites Polyester-Polyurethan-Elastomer* bezeichnet. Das zweite Polyester-Polyurethan-Elastomer kann also auch ein Gemisch verschiedener Polyester-Polyurethan-Elastomere mit einer von Naphthylen-1,5-diisocyanat verschiedenen Isocyanatkomponente sein.

Die *Polyesterkomponente* bezeichnet die Gesamtheit aller Polyesterbestandteile, durch deren Reaktion mit den Isocyanatgruppen der *Isocyanatkomponente* das Polyester-Polyurethan-Elastomer ursprünglich gebildet wurde, wobei mit *Isocyanatkomponente* die Gesamtheit der Isocyanate gemeint ist, die in der Herstellung des Polyester-Polyurethan-Elastomers Anwendung fanden.

Als *Carbamate* werden in der Terminologie der vorliegenden Erfindung die bei Durchführung der Chemolyse infolge der Reaktion mit dem Chemolysealkohol *gebildeten* Urethane bezeichnet, um sie von dem *eingesetzten* Urethan unterscheiden zu können. So wird beispielsweise das Reaktionsprodukt des NDI-Bausteins eines 1,5-NDI-basierten Polyester-Polyurethan-Elastomers mit Methanol als 1,5-NDI-Dimethylcarbamat bezeichnet. Diese Wahl der Begrifflichkeiten dient lediglich der Vereinfachung der Diskussion.

Es folgt zunächst eine Kurzzusammenfassung verschiedener möglicher Ausführungsformen der Erfindung:
In einer ersten Ausführungsform der Erfindung, die mit allen anderen Ausführungsformen außer solchen, die das Vorhandensein eines zweiten Polyester-Polyurethan-Elastomers zwingend vorsehen, kombiniert werden kann, enthält die Polymerzusammensetzung neben dem ersten Polyester-Polyurethan-Elastomer kein weiteres Polyester-Polyurethan-Elastomer.

In einer zweiten Ausführungsform der Erfindung, die mit allen anderen Ausführungsformen außer solchen, die das Vorhandensein eines zweiten Polyester-Polyurethan-Elastomers ausschließen, kombiniert werden kann, enthält die Polymerzusammensetzung ein zweites Polyester-Polyurethan-Elastomer, dessen Isocyanatkomponente kein Naphthylen-1,5-diisocyanat, aber Methylendiphenylendiisocyanat und/oder Toluylendiisocyanat umfasst (und insbesondere ist, d. h. keine weiteren Isocyanate umfasst, wobei das zweite Polyester-Polyurethan-Elastomer auch ein Gemisch verschiedener Polyester-Polyurethan-Elastomere mit einer von Naphthylen-1,5-diisocyanat verschiedenen Isocyanatkomponente umfassend Methylendiphenylendiisocyanat und/oder Toluylendiisocyanat sein kann).

In einer dritten Ausführungsform der Erfindung, die eine besondere Ausgestaltung der zweiten Ausführungsform ist, liegt der Massenanteil des ersten Polyester-Polyurethan-Elastomers, bezogen auf die Gesamtmasse des ersten und zweiten Polyester-Polyurethan-Elastomers, im Bereich von 60 % bis < 100 %, insbesondere 60 % bis 99 % oder 80 % bis 98 % oder 90 % bis 97% oder 95 % bis 96 %.

In einer vierten Ausführungsform der Erfindung, die eine besondere Ausgestaltung der zweiten und dritten Ausführungsform ist, enthält die Polymerzusammensetzung neben dem ersten und zweiten Polyester-Polyurethan-Elastomer kein weiteres Polyester-Polyurethan-Elastomer.

In einer fünften Ausführungsform der Erfindung, die eine besondere Ausgestaltung der zweiten, dritten und vierten Ausführungsform ist, basiert die Polyesterkomponente des zweiten Polyester-Polyurethan-Elastomers zumindest teilweise auf einer Säurekomponente und einer Alkoholkomponente (d. h. sie wurde durch Polykondensation derselben hergestellt), wobei
die Säurekomponente ausgewählt ist aus Adipinsäure, Bernsteinsäure, Therephthalsäure, Sebacinsäure oder einem Gemisch von zwei oder mehr derselben, und
die Alkoholkomponente ausgewählt ist aus Monoethylenglykol, Diethylenglykol oder einem Gemisch derselben.

In einer sechsten Ausführungsform der Erfindung, die eine besondere Ausgestaltung der zweiten, dritten, vierten und fünften Ausführungsform ist, basiert die Polyesterkomponente des zweiten Polyester-Polyurethan-Elastomers zumindest teilweise auf einer cyclischen Carbonsäureesterkomponente (d. h. sie wurde durch Ringöffnungspolymerisation derselben hergestellt), wobei die cyclische Carbonsäurekomponente ε-Caprolacton ist.

In einer siebten Ausführungsform der Erfindung, die eine besondere Ausgestaltung der zweiten, dritten, vierten, fünften und sechsten Ausführungsform ist, umfasst das zweite Polyester-Polyurethan-Elastomer neben Urethangruppen zusätzlich Harnstoffgruppen.

In einer achten Ausführungsform der Erfindung, die mit allen anderen Ausführungsformen kombiniert werden kann, basiert die Polyesterkomponente des ersten Polyester-Polyurethan-Elastomers zumindest teilweise auf einer Säurekomponente und einer Alkoholkomponente (d. h. sie wurde durch Polykondensation derselben hergestellt, wobei
die Säurekomponente ausgewählt ist aus Adipinsäure, Bernsteinsäure oder einem Gemisch derselben,
   und
die Alkoholkomponente der Polyesterkomponente des ersten Polyester-Polyurethan-Elastomers ausgewählt ist aus Monoethylenglykol, Diethylenglykol, 1,4-Butandiol oder einem Gemisch von zwei oder mehr derselben.

In einer neunten Ausführungsform der Erfindung, die mit allen anderen Ausführungsformen kombiniert werden kann, basiert die Polyesterkomponente des ersten Polyester-Polyurethan-Elastomers zumindest teilweise auf einer cyclischen Carbonsäureesterkomponente (d. h. sie wurde durch Ringöffnungspolymerisation derselben hergestellt), wobei die cyclische Carbonsäurekomponente ε-Caprolacton ist.

In einer zehnten Ausführungsform der Erfindung, die mit allen anderen Ausführungsformen kombiniert werden kann, umfasst das erste Polyester-Polyurethan-Elastomer neben Urethangruppen zusätzlich Harnstoffgruppen.

In einer elften Ausführungsform der Erfindung, die mit allen anderen Ausführungsformen kombiniert werden kann, ist der optional vorhandene Kettenverlängerer und/oder Vernetzer ausgewählt aus 1,4-Butandiol, 1,6-Hexandiol, Hydrochinon-bis-(2-hydroxyethyl)-ether, Trimethylolpropan (= 2-Ethyl-2-hydroxymethylpropan-1,3-diol), Triisopropanolamin, Thiodiglykol (= Bis(2-hydroxyethyl)sulfid) oder einem Gemisch aus zwei oder mehr derselben.

In einer zwölften Ausführungsform der Erfindung, die mit allen anderen Ausführungsformen kombiniert werden kann, liegt der Massenanteil an Polyester-Polyurethan-Elastomer (d. h. an der Gesamtheit aller vorhandenen Polyester-Polyurethan-Elastomere) in der Polymerzusammensetzung im Bereich von 60 % bis 100 %, bevorzugt 80 % bis 100 %, besonders bevorzugt 90 % bis 100 %, ganz besonders bevorzugt 95 % bis 100 %, außerordentlich ganz besonders bevorzugt 97 % bis 100 %, bezogen auf die Gesamtmasse der Polymerzusammensetzung.

In einer dreizehnten Ausführungsform der Erfindung, die eine besondere Ausgestaltung der zwölften Ausführungsform ist, umfasst ein nicht auf Polyester-Polyurethan-Elastomer zurückzuführender Anteil der Polymerzusammensetzung (insbesondere thermoplastische) Polyurethane (und besteht gegebenenfalls aus diesen), die auf einer Polyetherpolyol-umfassenden und/oder Polycarbonatpolyol-umfassenden Polyolkomponente (ohne Polyester-Bestandteile) und einer Methylendiphenylendiisocyanat-umfassenden und/oder Toluylendiisocyanat-umfassenden Isocyanatkomponente basieren.

In einer vierzehnten Ausführungsform der Erfindung, die mit allen anderen Ausführungsformen kombiniert werden kann, ist der lineare primäre Mono-Alkohol Methanol und/oder Ethanol, insbesondere Methanol.

In einer fünfzehnten Ausführungsform der Erfindung, die mit allen anderen Ausführungsformen kombiniert werden kann, werden der lineare primäre Mono-Alkohol und die Polymerzusammensetzung in Schritt (B) in einem Massenverhältnis

[m(linearer primärer Mono-Alkohol)/m(Polymerzusammensetzung)]

im Bereich von 10 bis 0,3, bevorzugt 5,0 bis 1,0 besonders bevorzugt 3,5 bis 2,5, eingesetzt.

In einer sechzehnten Ausführungsform der Erfindung, die mit allen anderen Ausführungsformen kombiniert werden kann, wird Schritt (B) bei einem Druck im Bereich von 10 bar bis 120 bar und einer Temperatur im Bereich von 130 °C bis 250 °C, bevorzugt 20 bar bis 60 bar und 170 °C bis 220 °C, besonders bevorzugt 25 bar bis 50 bar und 180 °C bis 210 °C, durchgeführt.

In einer siebzehnten Ausführungsform der Erfindung, die eine besondere Ausgestaltung der sechzehnten Ausführungsform ist, wird Schritt (B) beendet, indem das Produktgemisch entspannt und abgekühlt wird, wobei nicht umgesetzter linearer primärer Monoalkohol teilweise verdampft und nach Kondensation wieder in die Umsetzung von Schritt (B) zurückgeführt wird.

In einer achtzehnten Ausführungsform der Erfindung, die mit allen anderen Ausführungsformen außer solchen, die den Einsatz eines Katalysators in Schritt (B) zwingend vorsehen, kombiniert werden kann, wird Schritt (B) ohne Einsatz eines Katalysators durchgeführt.

In einer neunzehnten Ausführungsform der Erfindung, die mit allen anderen Ausführungsformen außer solchen, die den Einsatz eines Katalysators in Schritt (B) ausschließen, kombiniert werden kann, wird Schritt (B) in Gegenwart eines Katalysators durchgeführt, wobei der Katalysator (i) eine Alkylverbindung, eine Alkylhalogenidverbindung, ein Acetylacetonat, ein Carboxylat, ein Alkoxid, und/oder ein Chlorid eines Metalls der Gruppen 1, 4, 11, 12, 13 oder 14 des Periodensystems der Elemente und/oder (ii) ein (insbesondere tertiäres) Amin umfasst.

In einer zwanzigsten Ausführungsform der Erfindung, die eine besondere Ausgestaltung der neunzehnten Ausführungsform ist, wird der Katalysator ausgewählt aus der Gruppe bestehend aus einem Acetat (insbesondere Zn(OAc)₂), einem Übergangsmetallalkoholat (insbesondere Ti(OBu)₄ oder Bis[[1,1'-(butylimino-kN)bis[2-propanolato-kO]](2-)]-,(OC-6-21')-zinn, "Desmorapid 13-262 Dry"), einem Chlorid (insbesondere SnCl₂), einem tertiären Amin (insbesondere 4-Dimethylaminopyridin, DMAP), einem Acetylacetonat (insbesondere Titan(IV)acetylacetonat, "Tyzor AA 105") und einer Mischung aus zwei oder mehr der vorgenannten Katalysatoren.

In einer einundzwanzigsten Ausführungsform der Erfindung, die mit allen anderen Ausführungsformen kombiniert werden kann, umfasst Schritt (C):
Trennung der festen Carbamatphase von der flüssigen Alkoholphase, optional gefolgt von einer Wäsche sowie erforderlichenfalls von weiteren Reinigungsschritten der festen Carbamatphase, zur Isolierung des Carbamats.

In einer zweiundzwanzigsten Ausführungsform der Erfindung, die mit allen anderen Ausführungsformen, insbesondere der einundzwanzigsten, kombiniert werden kann, umfasst das Verfahren Folgendes:
(D) Weitere Umsetzung des in (C) isolierten Carbamats, wobei die weitere Umsetzung eine der folgenden umfasst:
(D.I) Hydrolyse des Carbamats mit Wasser in Gegenwart eines Hydrolyse-Katalysators unter Bildung von Naphthylen-1,5-diamin und des linearen primären Mono-Alkohols;
(D.II) Spalten des Carbamats in Naphthylen-1,5-diisocyanat und den linearen primären Mono-Alkohol;
(D.III) Hydrogenolyse des Carbamats mit Wasserstoff in Gegenwart eines Hydrogenolyse-Katalysators unter Bildung von Naphthylen-1,5-diamin und des linearen primären Mono-Alkohols;
   oder
(D.IV) Umsetzung des Carbamats mit einem Polyol in Gegenwart oder Abwesenheit eines Katalysators zu einem OH-terminierten Präpolymer.

In einer dreiundzwanzigsten Ausführungsform der Erfindung, die eine besondere Ausgestaltung der zweiundzwanzigsten Ausführungsform ist, umfasst der Hydrolyse-Katalysator eine (organische oder anorganische) Brønstedbase ausgewählt aus (i) einem Hydroxid (insbesondere Natriumhydroxid, Tetramethylammoniumhydroxid, Kaliumhydroxid oder Tetrabutylammoniumhydroxid), (ii) einem Carbonat (insbesondere ein Alkalimetallcarbonat wie zum Beispiel Natrium- oder Kaliumcarbonat), (iii) einem Hydrogencarbonat (insbesondere ein Alkalimetallhydrogencarbonat wie Natrium- oder Kaliumhydrogencarbonat) oder (iv) einem Orthophosphat oder Metaphosphat, bevorzugt Orthophosphat (insbesondere ein Alkalimetallphosphat oder Alkalimethallhydrogenphosphat) oder (v) einer Mischung aus zwei oder mehr der vorgenannten Brønstedbasen umfasst.

In einer vierundzwanzigsten Ausführungsform der Erfindung, die eine besondere Ausgestaltung der zweiundzwanzigsten und dreiundzwanzigsten Ausführungsform ist, umfasst der Hydrolyse-Katalysator eine Urethanase.

In einer fünfundzwanzigsten Ausführungsform der Erfindung, die eine besondere Ausgestaltung der vierundzwanzigsten Ausführungsform ist, wird als Urethanase eine der in EP 3 587 570 A1 beschriebenen Urethanasen verwendet.

In einer sechsundzwanzigsten Ausführungsform der Erfindung, die eine besondere Ausgestaltung der zweiundzwanzigsten Ausführungsform ist, wird das Spalten des Carbamats in (D.II) ohne Zusatz eines Carbamatspaltungs-Katalysators durchgeführt.

In einer siebenundzwanzigsten Ausführungsform der Erfindung, die eine weitere besondere Ausgestaltung der zweiundzwanzigsten Ausführungsform ist, wird das Spalten des Carbamats in (D.II) in Gegenwart eines Carbamatspaltungs-Katalysators durchgeführt.

In einer achtundzwanzigsten Ausführungsform der Erfindung, die eine besondere Ausgestaltung der siebenundzwanzigsten Ausführungsform ist, umfasst der Carbamatspaltungs-Katalysator
einen metallfreien oder metallhaltigen Brønsted- oder Lewis-sauren Katalysator
   oder
einen metallfreien oder metallhaltigen Brønsted- oder Lewis-basischen Katalysator.

In einer neunundzwanzigsten Ausführungsform der Erfindung, die eine besondere Ausgestaltung der zweiundzwanzigsten Ausführungsform ist, umfasst der Hydrogenolyse-Katalysator Kupfer, Palladium (insbesondere Pd/C, PdCl₂ oder Pd(OAc)₂), Nickel (insbesondere Raney-Nickel), Mangan (insbesondere Mn-Komplexe aufweisend einen dreizähnigen, über P- und N-Donoratome bindenden Chelatliganden sowie CO- und/oder Halogenliganden) oder Platin (insbesondere Platin(IV)-oxid).

In einer dreißigsten Ausführungsform der Erfindung, die eine besondere Ausgestaltung der zweiundzwanzigsten Ausführungsform ist, wird die Umsetzung des Carbamats mit dem Polyol in (D.IV) in Gegenwart eines Katalysators durchgeführt, der ein Carbonat, ein Hydrogencarbonat, ein Hydroxid, ein Orthophosphat, ein Mono-Hydrogen-Orthophosphat, ein Metaphosphat, ein Orthovanadat (wobei alle vorgenannten Katalysatoren vorzugsweise in Form ihrer Natrium- oder Kaliumsalze eingesetzt werden), ein Titanalkoholat (insbesondere Tetra-n-butyl-titanat, Ti(O nBu)4), ein tertiäres Amin (insbesondere 1,4-Diazabicyclo(2.2.2)octan, "DABCO"), Cäsiumfluorid, ein Stannat (insbesondere Dibutylzinndilaurat, "DBTL", oder Monobutylzinnoxid, n-Bu-Sn(O)OH, "MBTO") oder eine Mischung aus zwei oder mehr der vorgenannten Katalysatoren umfasst.

In einer einunddreißigsten Ausführungsform der Erfindung, die mit allen Ausführungsformen, die eine Gewinnung von Naphthylen-1,5-diamin vorsehen, kombiniert werden kann, wird das Naphthylen-1,5-diamin, gegebenenfalls nach einer Reinigung, insbesondere durch Umkristallisieren, (in an sich bekannter Weise) mit Phosgen zu Naphthylen-1,5-diisocyanat umgesetzt.

In einer zweiunddreißigsten Ausführungsform der Erfindung, die eine besondere Ausgestaltung der einundzwanzigsten und aller davon abgeleiteten Ausführungsformen ist, umfasst das Verfahren
(E.I.a) Extraktion der flüssigen Alkoholphase, optional nach destillativer Abtrennung von darin enthaltenem linearen primären Mono-Alkohol, mit einem organischen und/oder wässrigen Extraktionsmittel, das insbesondere ausgewählt ist aus (i) einem halogenierten, insbesondere aromatischen, Kohlenwasserstoff, optional in Verbindung mit Wasser, (ii) einem Kohlenwasserstoff, optional in Verbindung mit Wasser, (iii) einem primären Monoalkohol mit 6 bis 12 Kohlenstoffatomen, optional in Verbindung mit Wasser, (iv) einem Diol mit 2 bis 4 Kohlenstoffatomen, insbesondere Ethylenglykol, Propylenglykol, Diethylenglykol oder 1,4-Butandiol oder (v) Wasser, und Trennung in eine unpolare Phase und eine polare Phase;
(E.I.b) Optional, Verdampfung von gegebenenfalls vorhandenem Extraktionsmittel und/oder gegebenenfalls vorhandenem linearen primären Mono-Alkohol aus der unpolaren Phase unter Verbleib einer aufkonzentrierten organischen Phase;
(E.I.c) Umsetzung der unpolaren Phase oder (falls Schritt (E.I.b) durchgeführt wird) der aufkonzentrierten organischen Phase mit Wasserstoff in Gegenwart eines Hydrogenolyse-Katalysators unter Erhalt eines Alkohols (dessen Zusammensetzung von der Natur der Polyesterkomponente abhängt);
   und optional
(E.I.d) weitere Aufreinigung des Alkohols durch Destillation.

In einer dreiunddreißigsten Ausführungsform der Erfindung, die eine besondere Ausgestaltung der zweiunddreißigsten Ausführungsform ist, wird der Alkohol in der Synthese eines Polyesters eingesetzt.

In einer vierunddreißigsten Ausführungsform der Erfindung, die eine besondere Ausgestaltung der zweiunddreißigsten und dreiunddreißigsten Ausführungsform ist, wird die polare Phase zur Gewinnung einer Alkoholkomponente der Polyesterkomponente und/oder von optional vorhandenem Kettenverlängerer und/oder Vernetzer aufgearbeitet.

In einer fünfunddreißigsten Ausführungsform der Erfindung, die eine besondere Ausgestaltung der vierunddreißigsten Ausführungsform ist, wird/werden die aus der polaren Phase gewonnene Alkoholkomponente und/oder der aus der polaren Phase gewonnene Kettenverlängerer und/oder Vernetzer in der Herstellung neuen Polyester-Polyurethan-Elastomers eingesetzt.

In einer sechsunddreißigsten Ausführungsform der Erfindung, die - neben der zweiunddreißigsten Ausführungsform - eine weitere besondere Ausgestaltung der einundzwanzigsten und aller davon abgeleiteten Ausführungsformen ist, umfasst das Verfahren
(E.II.a) Umsetzung der flüssigen Alkoholphase mit Wasserstoff in Gegenwart eines Hydrogenolyse-Katalysators, wobei der Umsetzung mit Wasserstoff eine destillative Abtrennung von linearen primären Mono-Alkohol vorangeht und/oder folgt, sodass ein Gemisch aus (höher als der lineare primäre Mono-Alkohol siedenden) Alkoholen (dessen Zusammensetzung von der Natur der Polyesterkomponente abhängt) verbleibt;
   und
(E.II.b) destillative Trennung des Gemisches aus Alkoholen in einzelne Alkohol-Fraktionen.

In einer siebenunddreißigsten Ausführungsform der Erfindung, die eine besondere Ausgestaltung der sechsunddreißigsten Ausführungsform ist, werden eine oder mehrere der Alkohol-Fraktionen aus (E.II.b) in der Synthese eines Polyesters eingesetzt.

In einer achtunddreißigsten Ausführungsform der Erfindung, die eine besondere Ausgestaltung der zweiunddreißigsten bis siebenunddreißigsten Ausführungsform ist, umfasst der Hydrogenolyse-Katalysator Kupfer, Palladium (insbesondere Pd/C, PdCl₂ oder Pd(OAc)₂), Nickel (insbesondere Raney-Nickel) oder Platin (insbesondere Platin(IV)-oxid).

In einer neununddreißigsten Ausführungsform der Erfindung, die - neben der zweiunddreißigsten und sechsunddreißigsten Ausführungsform - eine weitere besondere Ausgestaltung der einundzwanzigsten und aller davon abgeleiteten Ausführungsformen ist, wird die in (C) erhaltene flüssige Alkoholphase in der Synthese eines Polyesters eingesetzt.

In einer vierzigsten Ausführungsform der Erfindung, die mit allen anderen Ausführungsformen kombiniert werden kann, umfasst Schritt (A) eine Trocknung der Polyester-Polyurethan-Elastomer-haltigen Polymerzusammensetzung mit Luft bei einer Temperatur von 80 °C bis 130 °C, wobei die zur Trocknung eingesetzte Luft bevorzugt eine relative Luftfeuchtigkeit von maximal 80 %, besonders bevorzugt von maximal 60 % aufweist, und wobei die Trocknung insbesondere solange durchgeführt wird, wie die absolute Luftfeuchtigkeit der Luft nach der Trocknung um 2 % oder mehr, bevorzugt um 1 % oder mehr, über der absoluten Luftfeuchtigkeit der zur Trocknung eingesetzten Luft liegt, wobei die absolute Luftfeuchtigkeit vor und nach der Trocknung jeweils mit einem Hygrometer des gleichen Typs (basierend auf dem gleichen Messprinzip) gemessen wird.

In einer einundvierzigsten Ausführungsform der Erfindung, die mit allen anderen Ausführungsformen kombiniert werden kann, enthält der lineare primäre Mono-Alkohol, bezogen auf seine Gesamtmasse, maximal 2,0 Massen-%, bevorzugt maximal 1,0 Massen-%, besonders bevorzugt maximal 0,50 Massen-% Wasser (bestimmt durch Karl-Fischer-Titration).

Die zuvor kurz geschilderten Ausführungsformen und weitere mögliche Ausgestaltungen der Erfindung werden im Folgenden näher erläutert. Sämtliche zuvor beschriebenen Ausführungsformen und die weiteren im Folgenden beschriebenen Ausgestaltungen der Erfindung sind, sofern sich aus dem Kontext für den Fachmann nicht eindeutig das Gegenteil ergibt oder nicht ausdrücklich etwas anderes gesagt wird, beliebig unter- und miteinander kombinierbar.

### BEREITSTELLUNG DER POLYMERZUSAMMENSETZUNG (SCHRITT (A))

In **Schritt (A)** des erfindungsgemäßen Verfahrens wird die wiederzuverwertende Polyester-Polyurethan-Elastomer-haltige Polymerzusammensetzung *bereitgestellt.*

Schritt (A) umfasst bevorzugt vorbereitende Schritte für die Chemolyse in Schritt (B). Dabei handelt es sich insbesondere um ein mechanisches Zerkleinern der Polymerzusammensetzung. Vorteilhaft ist ebenfalls eine Vorsortierung, die sicherstellt, dass die Polymerzusammensetzung, die dem nachfolgenden Schritt (B) zugeführt wird, möglichst keine störenden Begleitstoffe wie zum Beispiel von Polyester-Polyurethan-Elastomeren verschiedene Kunststoffe, metallische Verbindungen oder Verunreinigungen enthält.

Abhängig von der Beschaffenheit der zur Verfügung stehenden Polyester-Polyurethan-Elastomer-haltigen Polymerzusammensetzung kann es sinnvoll sein, diese vor der Alkoholyse in Schritt (B) zu trocknen. Dies kann beispielsweise so geschehen, dass man die, bevorzugt bereits mechanisch zerkleinerte, Polymerzusammensetzung mit Luft (insbesondere mit Luft einer relativen Luftfeuchtigkeit von maximal 80 %, bevorzugt von maximal 60 %) bei erhöhter Temperatur (bevorzugt von 80 °C bis 130 °C, beispielsweise bei 120 °C) solange behandelt, insbesondere durchströmt, bis der Wassergehalt der Polyester-Polyurethan-Elastomer-haltigen Polymerzusammensetzung hinreichend niedrig ist. Dies kann durch eine Bestimmung der absoluten Luftfeuchtigkeit (der "Wasserdampfdichte") der Abluft (der nach Kontakt mit der Polyester-Polyurethan-Elastomer-haltigen Polymerzusammensetzung austretenden Luft) kontrolliert werden. Beträgt die absolute Luftfeuchtigkeit in der Abluft allenfalls wenig mehr als in der zur Trocknung eingesetzten Luft, kann der Trocknungsvorgang beendet werden. Der Trocknungsvorgang wird insbesondere so lange durchgeführt, wie die absolute Luftfeuchtigkeit in der Abluft 2 % oder mehr, bevorzugt 1 % oder mehr, über der absoluten Luftfeuchtigkeit der zur Trocknung eingesetzten Luft liegt. Beträgt also die Luftfeuchtigkeit der zur Trocknung eingesetzten Luft x g_{Wasser}/m³_{Luft}, wird der Trocknungsvorgang so lange durchgeführt, wie die Luftfeuchtigkeit der Abluft einen Wert von (x + [*x* · 0,02]) g_{Wasser}/M³_{Luft} oder mehr aufweist. Dabei kann die Luftfeuchtigkeit mit handelsüblichen Hygrometern gemessen werden. Für die Messung vor und nach der Trocknung sollte der gleiche Hygrometer-Typ verwendet werden. Bevorzugt sind solche Hygrometer-Typen, die direkt die absolute Luftfeuchtigkeit angeben. Alternativ kann die absolute Luftfeuchtigkeit natürlich auch aus der von einem Hygrometer angegebenen relativen Luftfeuchtigkeit errechnet werden.

Bevorzugt besteht die dem Schritt (B) zuzuführende Polymerzusammensetzung, bezogen auf ihre Gesamtmasse, zu mindestens 60 % aus Polyester-Polyurethan-Elastomeren (unabhängig davon, ob 1,5-NDI-basiert oder nicht). Besonders bevorzugt beträgt der auf die Gesamtmasse der Polymerzusammensetzung bezogene Massenanteil an Polyester-Polyurethan-Elastomeren 80 % bis 100 %, ganz besonders bevorzugt 90 % bis 100 %, außerordentlich ganz besonders bevorzugt 95 % bis 100 % und insbesondere 97 % bis 100 %, wobei jeder dieser Wertebereiche mit jeder anderen Ausführungsform/ Ausgestaltungsmöglichkeit/Variante der Erfindung kombiniert werden kann. Der gegebenenfalls vorhandene nicht auf Polyester-Polyurethan-Elastomere zurückzuführende Anteil der Polymerzusammensetzung enthält bevorzugt (insbesondere thermoplastische) Polyurethane, die auf einer Polyetherpolyol-umfassenden und/oder Polycarbonatpolyol-umfassenden Polyolkomponente (ohne Polyester-Bestandteile) und einer Methylendiphenylendiisocyanat-umfassenden und/oder Toluylendiisocyanat-umfassenden Isocyanatkomponente basieren. Bevorzugt besteht der gegebenenfalls vorhandene nicht auf Polyester-Polyurethan-Elastomere zurückzuführende Anteil der Polymerzusammensetzung aus derartigen Polyurethanen.

Dabei kann es sich grundsätzlich um jede Art von Polyester-Polyurethan-Elastomer-haltiger Polymerzusammensetzung handeln, vorausgesetzt diese enthält mindestens ein Polyester-Polyurethan-Elastomer, dessen Isocyanatkomponente 1,5-NDI umfasst oder daraus besteht. Die Gesamtheit aller 1,5-NDI-basierten Polyester-Polyurethan-Elastomere, die in der Polymerzusammensetzung enthalten sind, wird im Rahmen der vorliegenden Erfindung als *erstes Polyester-Polyurethan-Elastomer* bezeichnet.

Was die Polyesterkomponente des ersten Polyester-Polyurethan-Elastomers betrifft, so ist es in einer Ausführungsform der Erfindung vorgesehen, dass diese auf einer Säurekomponente und einer Alkoholkomponente basiert (d. h. durch Polykondensation derselben hergestellt wurde), wobei
die Säurekomponente ausgewählt ist aus Adipinsäure, Bernsteinsäure oder einem Gemisch derselben,
   und
die Alkoholkomponente der Polyesterkomponente des ersten Polyester-Polyurethan-Elastomers ausgewählt ist aus Monoethylenglykol, Diethylenglykol, 1,4-Butandiol oder einem Gemisch von zwei oder mehr derselben.

Es ist natürlich ebenfalls möglich, dass die Polyesterkomponente des ersten Polyester-Polyurethan-Elastomers auf einer cyclischen Carbonsäureesterkomponente basiert (d. h. durch Ringöffnungspolymerisation derselben hergestellt wurde), wobei die cyclische Carbonsäurekomponente ε-Caprolacton ist.

Dem Fachmann ist bekannt, dass Polyester-Polyurethan-Elastomere wie das hier beschriebene erste Polyester-Polyurethan-Elastomer neben den Urethangruppen weitere funktionelle Gruppen wie insbesondere Harnstoffgruppen enthalten können. Dies verlässt den Rahmen der vorliegenden Erfindung selbstverständlich nicht.

In einer Ausführungsform der Erfindung umfasst die Polymerzusammensetzung neben dem ersten Polyester-Polyurethan-Elastomer kein weiteres Polyester-Polyurethan-Elastomer.

Es ist jedoch ebenfalls möglich, dass die Polymerzusammensetzung mindestens ein weiteres Polyester-Polyurethan-Elastomer enthält, dessen Isocyanatkomponente kein 1,5-NDI, aber Methylendiphenylendiisocyanat (fortan summarisch unabhängig von der Isomerenzusammensetzung: MDI) und/oder Toluylendiisocyanat (fortan summarisch unabhängig von der Isomerenzusammensetzung: TDI) umfasst (und insbesondere ist, d. h. keine weiteren Isocyanate umfasst). Die Gesamtheit aller in der Polymerzusammensetzung enthaltenen Polyester-Polyurethan-Elastomere, die nicht auf 1,5-NDI basieren, wird im Rahmen der vorliegenden Erfindung als *zweites Polyester-Polyurethan-Elastomer* bezeichnet. Das zweite Polyester-Polyurethan-Elastomer kann also auch eine Mischung verschiedener, nicht auf 1,5-NDI, sondern auf MDI und/oder TDI basierender Polyester-Polyurethan-Elastomere sein. Sollen Polymerzusammensetzungen wiederverwertet werden, die ein so definiertes erstes und zweites Polyester-Polyurethan-Elastomer enthalten, so wird bevorzugt durch eine geeignete Vorsortierung (siehe oben) sichergestellt, dass der Massenanteil des ersten Polyester-Polyurethan-Elastomers, bezogen auf die Gesamtmasse des ersten und zweiten Polyester-Polyurethan-Elastomers, im Bereich von 60 % bis < 100 %, insbesondere 60 % bis 99 % oder 80 % bis 98 % oder 90 % bis 97% oder 95 % bis 96 %, liegt. Es ist bevorzugt, dass die Polymerzusammensetzung neben dem ersten und zweiten Polyester-Polyurethan-Elastomer kein weiteres Polyester-Polyurethan-Elastomer enthält (d. h. es ist bevorzugt, dass keine Polyester-Polyurethan-Elastomere zugegen sind, die nicht auf 1,5-NDI, MDI und/oder TDI basieren).

Ebenso wie im Fall des ersten Polyester-Polyurethan-Elastomers kann auch die Polyesterkomponente des zweiten Polyester-Polyurethan-Elastomers auf einer Säurekomponente und einer Alkoholkomponente oder auf einer cyclischen Carbonsäureesterkomponente basieren. Im ersten Fall ist
die Säurekomponente bevorzugt ausgewählt aus Adipinsäure, Bernsteinsäure, Therephthalsäure, Sebacinsäure oder einem Gemisch von zwei oder mehr derselben,
   und
die Alkoholkomponente bevorzugt ausgewählt aus Monoethylenglykol, Diethylenglykol oder einem Gemisch derselben.

Im zweiten Fall ist die cyclische Carbonsäurekomponente insbesondere ε-Caprolacton. Selbstredend kann auch das zweite Polyester-Polyurethan-Elastomer neben Urethangruppen zusätzlich Harnstoffgruppen enthalten.

Für alle Polyester-Polyurethan-Elastomere gilt, dass der optional vorhandene Kettenverlängerer und/oder Vernetzer bevorzugt ausgewählt ist aus 1,4-Butandiol, 1,6-Hexandiol, Hydrochinon-bis-(2-hydroxyethyl)-ether, Trimethylolpropan (= 2-Ethyl-2-hydroxymethylpropan-1,3-diol), Triisopropanolamin, Thiodiglykol (= Bis(2-hydroxyethyl)sulfid) oder einem Gemisch aus zwei oder mehr derselben.

### UMSETZUNG DER DER POLYMERZUSAMMENSETZUNG MIT DEM LINEAREN PRIMÄREN MONO-ALKOHOL (SCHRITT (B))

In **Schritt (B)** des erfindungsgemäßen Verfahrens wird die in Schritt (A) bereitgestellte Polymerzusammensetzung mit einem (überstöchiometrisch eingesetzten) linearen primären Mono-Alkohol, dem Chemolysealkohol, umgesetzt (Chemolyse des Polymerrückgrats). Hierzu wird bevorzugt Methanol und/oder Ethanol, besonders bevorzugt Methanol, eingesetzt.

Dabei ist es bevorzugt, ein Massenverhältnis

[m(linearer primärer Mono-Alkohol)/m(Polymerzusammensetzung)]

von 10 bis 0,3, bevorzugt 5,0 bis 1,0 besonders bevorzugt 3,5 bis 2,5, einzuhalten. Es versteht sich von selbst, dass der eingesetzte lineare primäre Mono-Alkohol möglichst wenig Wasser enthalten sollte, um eine möglichst selektive Bildung des Carbamats zu ermöglichen. Insbesondere ist es bevorzugt, dass der eingesetzte lineare primäre Mono-Alkohol, bezogen auf seine Gesamtmasse, maximal 2,0 Massen-%, bevorzugt maximal 1,0 Massen-%, besonders bevorzugt maximal 0,50 Massen-% Wasser enthält, was erforderlichenfalls durch an sich bekannte Trocknungsmaßnahmen realisiert werden kann. Als Masse des linearen primären Mono-Alkohols gilt für die Zwecke der vorliegenden Erfindung in jedem Fall die Gesamtmasse inklusive gegebenenfalls vorhandenen Wassers. Der Wassergehalt des linearen primären Mono-Alkohols ist erforderlichenfalls durch Karl-Fischer-Titration bestimmbar; dies ist die für die Zwecke der vorliegenden Erfindung maßgebliche Methode. Die Karl-Fischer-Titration ist vielfach beschrieben und dem Fachmann wohlbekannt. Verschiedene Ausgestaltungsmöglichkeiten des Grundprinzips der Karl-Fischer-Titration ergeben im Allgemeinen im Rahmen für die Zwecke der vorliegenden Erfindung hinreichend gut übereinstimmende Ergebnisse. Im Zweifelsfall ist die Karl-Fischer-Titration wie in DIN 51 777, Teil 1, März 1983 beschrieben maßgeblich für die Zwecke der vorliegenden Erfindung.

Die Umsetzung in Schritt (B) erfolgt vorzugsweise bei einem Druck im Bereich von 10 bar bis 120 bar und einer Temperatur im Bereich von 130 °C bis 250 °C, besonders bevorzugt 20 bar bis 60 bar und 170 °C bis 220 °C, und ganz besonders bevorzugt 25 bar bis 50 bar und 180 °C bis 210 °C. Druckangaben sind hier und im Folgenden, sofern nicht ausdrücklich etwas anderes gesagt wird, als Absolutdrücke zu verstehen.

Zur Beendigung der Chemolyse wird bevorzugt so verfahren, dass das unter Druck stehende Produktgemisch entspannt (insbesondere auf Umgebungsdruck) und abgekühlt (insbesondere auf Umgebungstemperatur) wird, wobei nicht umgesetzter linearer primärer Monoalkohol teilweise verdampfen und nach Kondensation wieder in die Umsetzung von Schritt (B) zurückgeführt werden kann. Es ist selbstverständlich auch möglich, zuerst abzukühlen und dann zu entspannen; die Formulierung "*entspannt und abgekühlt wird"* soll nicht zwingend eine Reihenfolge implizieren. Wenn vor der Entspannung abgekühlt wird, ist der Anteil verdampften Chemolysealkohols entsprechend geringer. Die Umsetzung in Schritt (B) dauert von Erreichen der vorgesehenen Reaktionstemperatur bis zum Erreichen einer Temperatur unterhalb des Normalsiedepunktes (= Siedetemperatur bei Normaldruck, also bei 1013,25 hPa) des verwendeten Chemolysealkohols bevorzugt 1,0 h bis 10 h, besonders bevorzugt 3,0 h bis 8,0 h, ganz besonders bevorzugt 4,0 h bis 6,0 h

Die Chemolyse von Schritt (B) kann sowohl in Gegenwart als auch in Abwesenheit eines Katalysators durchgeführt werden. Wird ein Katalysator eingesetzt, wird dieser bevorzugt ausgewählt aus (i) einer Alkylverbindung, einer Alkylhalogenidverbindung, einem Acetylacetonat, einem Carboxylat, einem Alkoxid, und/oder einem Chlorid eines Metalls der Gruppen 1, 4, 11, 12, 13 oder 14 des Periodensystems der Elemente und/oder (ii) einem, insbesondere tertiärem, Amin. In einer besonders bevorzugten Ausführungsform wird als Katalysator ein Acetat (insbesondere Zn(OAc)₂), ein Übergangsmetallalkoholat (insbesondere Ti(OBu)₄ oder Bis[[1,1'-(butylimino-kN)bis[2-propanolato-kO]](2-)]-,(OC-6-21')-zinn, "Desmorapid 13-262 Dry"), ein Chlorid (insbesondere SnCl₂), ein tertiäres Amin (insbesondere 4-Dimethylaminopyridin, DMAP), ein Acetylacetonat (insbesondere Titan(IV)acetylacetonat, "Tyzor AA 105") oder eine Mischung aus zwei oder mehr der vorgenannten Katalysatoren eingesetzt.

Als Reaktoren für Schritt (B) eignen sich grundsätzlich alle in der Fachwelt für einen derartigen Zweck (Chemolyse) bekannten Reaktoren. Insbesondere geeignet sind Rührkessel (gerührte Reaktoren; chargenweise, kontinuierlich oder semikontinuierlich betrieben) und Rohrreaktoren (kontinuierlich durchströmte Reaktionsrohre, wobei auch mehrere zu einem sog. Rohrbündelreaktor verbunden sein können).

Die Chemolyse liefert ein Produktgemisch umfassend
eine flüssige Alkoholphase (enthaltend (i) nicht umgesetzten linearen primären Mono-Alkohol, (ii) Produkte der Alkoholyse der Urethanbindungen und Esterbindungen, und (iii) gegebenenfalls Kettenverlängerer und/oder Vernetzer);
   und
eine feste Carbamatphase (enthaltend ein (Mono- und/oder Bis-, insbesondere Bis-)Carbamat des Naphthylen-1,5-diisocyanats und des linearen primären Mono-Alkohols).

Es ist möglich und verlässt den Rahmen der vorliegenden Erfindung nicht, dass das Produktgemisch weitere Bestandteile neben den genannten enthält, beispielsweise geringe Anteile an Naphthylen-1,5-diamin (fortan: 1,5-NDA) insbesondere in der festen Phase. Infolge der vorliegenden Löslichkeitsgleichgewichte ist natürlich ebenfalls möglich, dass die genannten Bestandteile nicht zu 100 % in der flüssigen *oder* festen Phase vorliegen; so können beispielsweise geringe Mengen der gegebenenfalls vorhandenen Kettenverlängerer und/oder Vernetzer in die feste Carbamatphase gelangen (darin "eingeschlossen" werden). Derartiges verlässt den Rahmen der vorliegenden Erfindung selbstverständlich nicht. Enthält die ursprüngliche Polymerzusammensetzung auch das zweite Polyester-Polyurethan-Elastomer, so werden auch MDI- und/oder TDI-Carbamate erhalten. Diese gelangen überwiegend bis vollständig in die flüssige Alkoholphase. Sollten geringfügige Anteile davon in die feste Carbamatphase gelangen, können diese (oder, abhängig von der weiteren Umsetzung der Carbamate, deren Folgeprodukte) durch übliche Reinigungsmethoden abgetrennt werden.

### ISOLIEREN DES CARBAMATS AUS DEM PRODUKTGEMISCH (SCHRITT (C))

In **Schritt (C)** des erfindungsgemäßen Verfahrens wird das Carbamat aus dem Produktgemisch isoliert, damit es einer weiteren Verwendung zugeführt werden kann.

Im einfachsten Fall umfasst Schritt (C) lediglich die Trennung der festen Carbamatphase von der flüssigen Alkoholphase durch eine Fest-flüssig-Phasentrennung, insbesondere durch Filtration oder Zentrifugation. Hieran können sich eine Wäsche sowie erforderlichenfalls weitere Reinigungsschritte anschließen. Es ist beispielsweise möglich, die feste Carbamatphase in einem gegenüber Carbamaten inerten organischen Lösungsmittel umzukristallisieren, um etwa gegebenenfalls vorhandene Anteile an MDI-und/oder TDI-Carbamaten (oder anderen mitgeschleppten Begleitstoffen) abzutrennen. Eine Umkristallisation ist insbesondere auch dann vorteilhaft, wenn die in Schritt (A) bereitgestellte Polymerzusammensetzung andere, nicht auf Polyester-Polyurethan-Elastomere zurückzuführende Bestandteile enthält. Sind beispielsweise Anteile an thermoplastischen Polyurethanen auf Polyetherpolyolbasis vorhanden, können solche Polyetherpolyole zumindest teilweise auch die Carbamatphase gelangen. Nach Abtrennung in einer Umkristallisation können diese wiederverwendet oder, sollte sich eine stoffliche Verwendung mengenmäßig nicht lohnen, verbrannt werden, vorzugsweise unter energetischer Nutzung der Verbrennungswärme.

### WEITERE UMSETZUNG DES CARBAMATS (SCHRITT (D))

Das auf diese Weise gewonnene Carbamat wird bevorzugt in einem **Schritt (D)** weiter umgesetzt. Geeignete weitere Umsetzungen sind dabei insbesondere:
(D.I) **Hydrolyse des Carbamats** mit Wasser in Gegenwart eines Hydrolyse-Katalysators unter Bildung von Naphthylen-1,5-diamin und des linearen primären Mono-Alkohols;
(D.II) **Spalten des Carbamats** in Naphthylen-1,5-diisocyanat und den linearen primären Mono-Alkohol;
(D.III) **Hydrogenolyse des Carbamats** mit Wasserstoff in Gegenwart eines Hydrogenolyse-Katalysators unter Bildung von Naphthylen-1,5-diamin und des linearen primären Mono-Alkohols,
   oder
(D.IV) **Umsetzung des Carbamats** mit einem Polyol in Gegenwart oder Abwesenheit eines Katalysators **zu einem OH-terminierten Präpolymer.**

Die *Hydrolyse,* die *Spaltung* und die *Hydrogenolyse* von Carbamaten sowie ihre *Umsetzung (Umurethanisierung) mit Polyolen* sind grundsätzlich in der Fachwelt bekannte Reaktionen und werden an dieser Stelle daher nur kurz umrissen.

Die **Hydrolyse gemäß (D.I)** liefert das zu dem Isocyanat der Isocyanatkomponente korrespondierende Amin, also Naphthylen-1,5-diamin. Sollten bei der Hydrolyse noch Anteile von anderen Carbamaten, insbesondere Anteile von MDI- und/oder TDI-Carbamaten, zugegen sein, werden diese natürlich ebenfalls zu den entsprechenden Aminen hydrolysiert. Aufgrund der vergleichsweise geringen Wasserlöslichkeit von Naphthylen-1,5-diamin im Vergleich zu anderen Aminen wie beispielsweise den Isomeren des Toluylendiamins (TDA), ist es möglich, 1,5-NDA nach der Hydrolyse als Feststoff zu isolieren. Sollte die Reinheit des Feststoffs nach der Separation nicht ausreichend hoch sein, kann diese beispielsweise durch Umkristallisation in einem organischen Lösungsmittel (z. B. Monochlorbenzol) erhöht werden. Das Umkristallisieren kann zudem genutzt werden, um bis hierhin mitgeschleppte feste Verunreinigungen aus der Polymerzusammensetzung abzutrennen. Hierbei wird die feste Aminphase bei erhöhter Temperatur (z. B. 120 °C) und einem Druck von bis zu 4 bar (absolut) im organischen Lösungsmittel gelöst und per Filtration und/oder Zentrifugation von den ungelösten Feststoffen abgetrennt. Anschließend wird das 1,5-NDA durch Temperreduktion kristallisiert und mittels Filtration/Zentrifugation vom organischen Lösungsmittel abgetrennt. Das organische Lösungsmittel kann bevorzugt über Destillation aufgereinigt und dadurch größtenteils im Kreislauf geführt werden. Ein Teilstrom des organischen Lösungsmittels kann zudem zum Waschen der festen Aminphase nach der Hydrolyse der Carbamate verwendet werden.

Grundsätzlich kann eine derartige Hydrolyse analog zur direkten Hydrolyse von Polyurethanen durchgeführt werden (siehe hierzu die weiter oben zitierte Literatur, insbesondere der Übersichtsartikel [1]). Die so gewonnenen Amine (1,5-NDA sowie gegebenenfalls andere Amine wie insbesondere MDA und/oder TDA) können dann allen in der Fachwelt für derartige Amine bekannten Verwendungszwecken zugeführt werden, u. a. können sie wieder zu den korrespondierenden Isocyanaten phosgeniert werden. Ein so gewonnenes Isocyanat kann dann wieder der Herstellung eines Polyurethans, insbesondere eines Polyester-Polyurethan-Elastomers, zugeführt werden. Die Hydrolyse kann durch Einsatz eines Hydrolyse-Katalysators unterstützt werden. Hierzu eignet sich insbesondere:
(I) eine (organische oder anorganische) Brønstedbase ausgewählt aus (i) einem Hydroxid (insbesondere Natriumhydroxid, Tetramethylammoniumhydroxid, Kaliumhydroxid oder Tetrabutylammoniumhydroxid), (ii) einem Carbonat (insbesondere ein Alkalimetallcarbonat wie zum Beispiel Natrium- oder Kaliumcarbonat), (iii) einem Hydrogencarbonat (insbesondere ein Alkalimetallhydrogencarbonat wie Natrium- oder Kaliumhydrogencarbonat), (iv) einem Orthophosphat oder Metaphosphat, bevorzugt Orthophosphat (insbesondere ein Alkalimetallphosphat oder Alkalimethallhydrogenphosphat) oder (v) eine Mischung aus zwei oder mehr der vorgenannten Brønstedbasen,
   und/oder
(II) eine Urethanase, insbesondere eine der in EP 3 587 570 A1 beschriebenen Urethanasen.

Es ist jedoch auch denkbar, das Isocyanat der Isocyanatkomponente direkt aus dem Carbamat zu gewinnen, indem das Carbamat - entweder rein thermisch oder in Gegenwart eines Carbamatspaltungs-Katalysators - in das Isocyanat der Isocyanatkomponente und den Chemolysealkohol gespalten wird **(Carbamat-Spaltung gemäß (D.II)).** Bei katalytischer Durchführung eignet sich als Carbamatspaltungs-Katalysator insbesondere:
(I) ein metallfreier oder metallhaltiger Brønsted- oder Lewis-saurer Katalysator oder
(II) ein metallfreier oder metallhaltiger Brønsted- oder Lewis-basischer Katalysator.

Für weitere Details sei auf W. Leitner et al., Carbon2Polymer - Chemical Utilization of CO2 in the Production of Isocyanates, Kapitel 4, "Carbamate Cleavage", erschienen in Chem. Ing. Tech. 2018, 90, 1504 - 1512 und die darin zitierte Literatur verwiesen.

Die Aufarbeitungsvariante gemäß (D.II) liefert direkt das Isocyanat der Isocyanatkomponente, sodass eine erneute Phosgenierung überflüssig ist. Das Isocyanat der Isocyanatkomponente ist mindestens 1,5-NDI. Sollten andere Carbamate, insbesondere MDI- und/oder TDI-Carbamate, anteilig mit in die feste Carbamatphase gelangt und nicht bereits vorher abgetrennt worden sein, werden diese auch in die entsprechenden Isocyanate überführt. Eine Trennung verschiedener Isocyanate ist gegebenenfalls durch an sich bekannte Techniken (Umkristallisation, Destillation) ohne weiteres möglich.

Das zu dem Isocyanat der Isocyanatkomponente korrespondierende Amin, also mindestens wie bei (D.I) beschrieben 1,5-NDA, kann auch durch die **Hydrogenolyse des Carbamats gemäß (D.III)** mit Wasserstoff erhalten werden. Ein Verfahren, das direkt von Polyurethanen ausgeht und auch für den vorliegenden Schritt (D.II) anwendbar ist, ist in *Hydrogenative Depolymerization of Polyurethanes Catalyzed by Manganese Pincer Complex* von Viktoriia Zubar et al., erschienen in ChemSusChem 2022, 15, e202101606 [2], beschrieben. Es sei ebenfalls auf die in [2] zitierte Literatur verwiesen. Die Verwendungsmöglichkeiten des Amins sind die gleichen wir für (D.I) geschildert. Die Hydrogenolyse wird bevorzugt durch Einsatz eines Katalysators unterstützt. Als Hydrogenolyse-Katalysator eignet sich insbesondere ein Katalysator, der
Palladium (insbesondere Pd/C, PdCl₂ oder Pd(OAc)₂), Kupfer, Nickel (insbesondere Raney-Nickel), Mangan (insbesondere Mn-Komplexe aufweisend einen dreizähnigen, über P-und N-Donoratome bindenden Chelatliganden sowie CO- und/oder Halogenliganden) oder Platin (insbesondere Platin(IV)-oxid) umfasst.

Die Aufarbeitungsvarianten gemäß (D.I) und (D.III) liefern also das zum Isocyanat der Isocyanatkomponente korrespondierende Amin (hier mindestens 1,5-NDA), welches wiederum, gegebenenfalls nach einer Reinigung, insbesondere durch Umkristallisieren, allen in der Fachwelt bekannten Verwendungszwecken zugeführt werden kann. Insbesondere kann das Amin in an sich bekannter Weise mit Phosgen zum korrespondierenden Isocyanat (hier also mindestens zu 1,5-NDI) umgesetzt werden und in der Herstellung neuen Polyurethans, insbesondere neuen Polyester-Polyurethan-Elastomers, eingesetzt werden.

Die **Umsetzung des Carbamats aus Schritt (C) mit einem Polyol zu einem OH-terminierten Präpolymer gemäß (D.IV)** ist eine weitere Möglichkeit der Weiterverarbeitung des Carbamats. Chemisch handelt es sich um eine Umurethanisierung und damit prinzipiell um die gleiche Art von Reaktion, die auch der Glykolyse von Polyurethanen zugrunde liegt (siehe hierzu die weiter oben zitierte Literatur, insbesondere der Übersichtsartikel [1]). Hierfür wird das Carbamat mit einem Polyol umgesetzt, wobei die OH-Gruppen des Polyols stöchiometrisch oder überstöchiometrisch, insbesondere leicht überstöchiometrisch (z. B. 5 bis 10 % Überschuss auf molarer Basis), zu den vorhanden Carbamat-Funktionalitäten eingesetzt werden. Diese Umsetzung führt zu **OH-terminierten Präpolymeren.** Diese können allen in der Fachwelt bekannten Verwendungszwecken zugeführt werden, sie können insbesondere in einer Reaktion mit Isocyanaten als Präpolymere für Elastomere, Weichschaum- und Hartschaumanwendungen, thermoplastische Polyurethane, Coatings und Klebstoffe eingesetzt werden.

Das für Schritt (D.IV) eingesetzte Polyol hat einen Siedepunkt, der über dem des eingesetzten Chemolysealkohols liegt. In einer besonders bevorzugten Ausführungsform wird der Chemolysealkohol während der Reaktion mit dem Polyol kontinuierlich destillativ aus dem Reaktionsgemisch entfernt. Die Reaktion kann optional in Gegenwart eines Katalysators durchgeführt werden. Dieser umfasst bevorzugt ein Carbonat, ein Hydrogencarbonat, ein Hydroxid, ein Orthophosphat, ein Mono-Hydrogen-Orthophosphat, ein Metaphosphat, ein Orthovanadat (wobei alle vorgenannten Katalysatoren vorzugsweise in Form ihrer Natrium- oder Kaliumsalze eingesetzt werden), ein Titanalkoholat (insbesondere Tetra-*n*-butyl-titanat, Ti(O-*n*Bu)₄), ein tertiäres Amin (insbesondere 1,4-Diazabicyclo(2.2.2)octan, "DABCO"), Cäsiumfluorid, ein Stannat (insbesondere Dibutylzinndilaurat, "DBTL", oder Monobutylzinnoxid, n-Bu-Sn(O)OH, "MBTO") oder eine Mischung aus zwei oder mehr der vorgenannten Katalysatoren.

Typische geeignete Polyole sind **zweiwertige Polyole** (insbesondere Ethylenglykol, Diethylenglykol, Propylenglykol, Dipropylenglykol, 1,3-Propandiol, 1,4-Butandiol, 1,4-Butendiol, 1,4-Butindiol, Neopentylglykol, 1,5-Pentandiol, Methylpentandiole (wie beispielweise 3-Methyl-1,5-pentandiol), 1,6-Hexandiol, 1,8-Octandiol, 1,10-Decandiol, 1,12-Dodecandiol, Bis-(hydroxymethyl)-cyclohexane (wie beispielweise 1,4-Bis-(hydroxymethyl)cyclohexan), Triethylenglykol, Tetraethylenglykol, Polyethylenglykole, Dipropylenglykol, Tripropylenglykol, Polypropylenglykole, Dibutylenglykol, Polybutylenglykole und Polyesterpolyole, z. B. aus Ethylenglykol und Adipinsäure), **dreiwertige Polyole** (insbesondere Trimethylolpropan, Glycerin, Trishydroxyethylisocyanurat), **vierwertige Polyole** (insbesondere Pentaerythrit) und **Polyole, die aus nachwachsenden Rohstoffen gewonnen werden können** (insbesondere Sorbit, Hexit, Saccharose, Stärke, Stärkehydrolysate, Cellulose, Cellulosehydrolysate und hydroxyfunktionalisierte Fette und Öle, insbesondere Rizinusöl), sowie alle **Modifizierungsprodukte** dieser zuvor genannten Polyole mit unterschiedlichen Mengen an ε-Caprolacton. Als Polyole für Schritt (D.IV) können ebenfalls Polyetherpolyole eingesetzt werden, insbesondere solche, die eine gemäß DIN55672-1 (2016-03) bestimmte zahlengemittelte Molmasse Mₙ im Bereich von 18 g/mol bis 8000 g/mol und eine (aus den in der Herstellung der Polyetherpolyole eingesetzten H-funktionellen Startern berechnete) Funktionalität von 2 bis 3 aufweisen. Bevorzugt sind Polyetherpolyole, die aus sich wiederholenden Ethylenoxid- und Propylenoxideinheiten aufgebaut sind, bevorzugt mit einem Anteil von 35 % bis 100 % Propylenoxideinheiten, besonders bevorzugt mit einem Anteil von 50 % bis 100 % Propylenoxideinheiten. Hierbei kann es sich um statistische Copolymere, Gradienten-Copolymere, alternierende oder Blockcopolymere aus Ethylenoxid und Propylenoxid handeln.

In allen vier Varianten wird der eingesetzte Chemolysealkohol wieder freigesetzt und kann durch Destillation zurückgewonnen und wieder in der Chemolyse eingesetzt werden.

### WEITERVERARBEITUNG DER FLÜSSIGEN ALKOHOLPHASE (SCHRITT (E))

Die in Schritt (C) nach Isolierung der festen Carbamatphase anfallende flüssige Alkoholphase wird bevorzugt in einem **Schritt (E)** weiterverarbeitet. Diese flüssige Alkoholphase umfasst mindestens nicht umgesetzten Chemolysealkohol und die in Schritt (B) gebildeten Abbauprodukte des dem Polyester-Polyurethan-Elastomers zugrunde liegenden Polyesters. Hierbei handelt es sich, abhängig von den in der Herstellung des Polyesters eingesetzten Ausgangskomponenten, um (i) *Ester der Säurekomponente und des Chemolysealkohols* (z. B. Adipinsäuremethylester, fortan: ADM) sowie um die *Alkoholkomponente* (z. B. Monoethylenglykol) bzw. um (ii) *offenkettige Ester der cyclischen Carbonsäurekomponente* (z. B. 6-Hydroxy-Hexansäuremethylester). Wurden in der Herstellung des Polyester-Polyurethan-Elastomers auch Kettenverlängerer und/oder Vernetzer eingesetzt, werden diese in Schritt (B) wieder freigesetzt und gelangen ebenfalls in die flüssige Alkoholphase.

Im Fall von Polymerzusammensetzungen, die neben 1,5-NDI-basierten Polyester-Polyurethan-Elastomeren ("erstes Polyester-Polyurethan-Elastomer") auch andere Polyester-Polyurethan-Elastomere ("zweites Polyester-Polyurethan-Elastomer"), insbesondere MDI- und/oder TDI-basierte Polyester-Polyurethan-Elastomere enthalten, gelangen deren Abbauprodukte, also "nicht-NDI-Carbamate", insbesondere MDI-und/oder TDI-Carbamate, aufgrund ihrer im Vergleich zu den Carbamaten des 1,5-NDI besseren Löslichkeit überwiegend in die flüssige Alkoholphase und werden mit dieser weiterverarbeitet. Gleiches gilt für geringe lösliche Anteile der 1,5-NDI-Carbamate.

In einer **ersten Variante** umfasst Schritt (E) die folgende Abfolge an Schritten:
(E.I.a) Extraktion der flüssigen Alkoholphase, optional nach destillativer Abtrennung von darin enthaltenem linearen primären Mono-Alkohol, mit einem organischen und/oder wässrigen Extraktionsmittel, das insbesondere ausgewählt ist aus (i) einem halogenierten, insbesondere aromatischen, Kohlenwasserstoff, optional in Verbindung mit Wasser, (ii) einem Kohlenwasserstoff, optional in Verbindung mit Wasser, (iii) einem primären Monoalkohol mit 6 bis 12 Kohlenstoffatomen, optional in Verbindung mit Wasser, (iv) einem Diol mit 2 bis 4 Kohlenstoffatomen, insbesondere Ethylenglykol, Propylenglykol, Diethylenglykol oder 1,4Butandiol oder (v) Wasser, und Trennung in eine unpolare Phase und eine polare Phase;
(E.I.b) Optional, Verdampfung von gegebenenfalls vorhandenem Extraktionsmittel und/oder gegebenenfalls vorhandenem linearen primären Mono-Alkohol aus der unpolaren Phase unter Verbleib einer aufkonzentrierten organischen Phase;
(E.I.c) Umsetzung der unpolaren Phase oder (falls Schritt (E.I.b) durchgeführt wird) der aufkonzentrierten organischen Phase mit Wasserstoff in Gegenwart eines Hydrogenolyse-Katalysators unter Erhalt eines Alkohols (dessen Zusammensetzung von der Natur der Polyesterkomponente abhängt);
   und optional
(E.I.d) weitere Aufreinigung des Alkohols durch Destillation.

Das Ziel der *Extraktion* in Schritt (E.I.a) besteht darin, eine unpolare Phase und eine polare Phase zu erhalten, die sich voneinander trennen lassen und somit eine (Vor-)Trennung der Bestandteile der Alkoholphase möglich wird. So geht etwa ADM überwiegend in die unpolare Phase über. Dies kann erreicht werden, indem ein vergleichsweise unpolares Extraktionsmittel eingesetzt wird, in dem sich ADM löst, oder indem ein vergleichsweise polares Extraktionsmittel eingesetzt wird, das die eher polaren Bestandteile der Alkoholphase "herauslöst" und eine ADM-reiche, unpolare Phase zurücklässt. Die unpolare Phase enthält also typischerweise die Ester der Säurekomponente, ggf. gelöst in den unpolaren Bestandteilen des Extraktionsmittels. Sind in der Alkoholphase aus Schritt (C) auch Carbamate enthalten (1,5-NDI-Carbamate und andere Carbamate, insbesondere MDI-und/oder TDI-Carbamate), so lösen sich diese ebenfalls typischerweise in der unpolaren Phase. Die polare Phase enthält typischerweise die Alkoholkomponente- des Polyesters sowie Kettenverlängerer/Vernetzer gelöst im Chemolysealkohol (sofern dieser nicht vorher verdampft wurde) und ggf. die polaren Bestandteile des Extraktionsmittels.

Die in der unpolaren Phase gelösten Verbindungen, also insbesondere die Ester der Säurekomponente, werden in Schritt (E.I.c) einer *Hydrogenolyse* unterzogen, optional nach Verdampfung flüchtiger Bestandteile in einem Schritt (E.I.b). Als Katalysatoren für die Hydrogenolyse eignen sich die gleichen wie zuvor für Schritt (D.III) beschrieben. Durch die Hydrogenolyse wird der Ester der Säurekomponente in einen Alkohol überführt (der natürlich im Gemisch mit bei der Hydrogenolyse ebenfalls freigesetztem Chemolysealkohol aus der gespaltenen Esterbindung anfällt). Basiert also beispielsweise der Polyester des Polyester-Polyurethan-Elastomers auf der Säurekomponente Adipinsäure und wird in Schritt (B) als Chemolysealkohol Methanol eingesetzt, so bildet sich Adipinsäuremethylester, der über die Alkoholphase in die Hydrogenolyse gemäß Schritt (E.I.c) gelangt und dort in 1,6-Hexandiol und Methanol gespalten wird. Gegebenenfalls vorhandene Anteile an 6-Hydroxy-Hexansäuremethylester (aus anteilig vorhandenen auf ε-Caprolacton basierenden Polyesterkomponenten) werden in diesem Schritt zu 1,6-Hexandiol umgesetzt.

Bevorzugt schließt sich eine Destillation zur Reinigung des Alkohols an (Schritt (E.I.d). Gegebenenfalls an dieser Stelle noch vorhandenes Extraktionsmittel wird hierbei ebenso wie der Chemolysealkohol (der bei der Hydrogenolyse freigesetzt bzw. ggf. bis in die Hydrogenolyse mitgeschleppt wurde) destillativ entfernt. In der flüssigen Alkoholphase aus Schritt (C) gelöste Carbamate werden in der Hydrogenolyse zu den entsprechenden Aminen umgesetzt und verbleiben im Sumpf der Destillation. Ob eine stoffliche Verwertung dieser Amine sinnvoll ist, hängt von den Randbedingungen, insbesondere der Menge, in welcher diese anfallen, ab. Enthält die in Schritt (A) bereitgestellte Polymerzusammensetzung substanzielle Anteile an zweitem Polyester-Polyurethan-Elastomer und gelangen infolge dessen vergleichsweise große Mengen an MDI- und/oder TDI-Carbamaten in die Hydrogenolyse gemäß Schritt (E.I.c), kann es sinnvoll sein, die daraus entstandenen Amine weiterzuverarbeiten, insbesondere von gegebenenfalls ebenfalls vorhandenen Anteilen an 1,5-NDA zu trennen und weiter zu reinigen. Auf diese Weise gewonnene Amine können wie weiter oben bei Schritt (D.I) beschrieben verwendet werden. Es ist jedoch auch vorstellbar, dass Amine nur in einer solchen Menge anfallen, die eine stoffliche Wiederverwertung nicht sinnvoll erscheinen lassen. In diesem Fall ist es bevorzugt, die Amine zu verbrennen und die freiwerdende Verbrennungswärme energetisch zu nutzen.

Der in der Hydrogenolyse gewonnene Alkohol kann allen in der Fachwelt für derartige Alkohole bekannten Verwendungszwecken zugeführt werden, er kann insbesondere in der Synthese neuen Polyesters eingesetzt werden.

Bevorzugt wird ebenfalls die polare Phase aufgearbeitet, um die darin enthaltene Alkoholkomponente der Polyesterkomponente und/oder optional vorhandenen Kettenverlängerer/Vernetzer wiederzugewinnen. Hierzu können die Bestandteile der polaren Phase zunächst durch Destillation isoliert werden. Die so gewonnene Alkoholkomponente und/oder der so gewonnene Kettenverlängerer bzw. Vernetzer kann allen in der Fachwelt für derartige Verbindungen bekannten Verwendungszwecken zugeführt werden, er kann insbesondere in der Synthese neuen Polyester-Polyurethan-Elastomers eingesetzt werden.

In einer **zweiten Variante** umfasst der Schritt (E) folgende Schritte:
(E.II.a) Umsetzung der flüssigen Alkoholphase mit Wasserstoff in Gegenwart eines Hydrogenolyse-Katalysators, wobei der Umsetzung mit Wasserstoff eine destillative Abtrennung von linearen primären Mono-Alkohol vorangeht und/oder folgt, sodass ein Gemisch aus (höher als der lineare primäre Mono-Alkohol siedenden) Alkoholen (dessen Zusammensetzung von der Natur der Polyesterkomponente abhängt) verbleibt;
   und
(E.II.b) destillative Trennung des Gemisches aus Alkoholen in einzelne Alkohol-Fraktionen.

In dieser Variante wird die flüssige Alkoholphase ohne extraktive Vortrennung einer Hydrogenolyse (Schritt (E.II.a)) unterzogen. Als Katalysatoren eignen sich wiederum die gleichen, wie für Schritt (D.III) beschrieben.

Der Chemolysealkohol kann vor oder nach der Hydrogenolyse abgetrennt werden (ebenso wie gegebenenfalls vorhandenes Wasser). Bei dieser Verfahrensweise umfasst das Produkt der Hydrogenolyse neben dem durch die hydrogenolytische Spaltung gebildeten Alkohol (z. B. 1,6-Hexandiol) und dem Chemolysealkohol (mindestens der Anteil, der in der Hydrogenolyse freigesetzt wurde) noch die Alkoholkomponente (z. B. Monoethylenglykol) und ggf. Kettenverlängerer bzw. Vernetzer sowie gegebenenfalls Amine (gebildet aus in der flüssigen Alkoholphase gelösten Carbamaten). Aus diesem mindestens Alkohole umfassenden Gemisch werden durch Destillation (Schritt (E.II.b)) einzelne Alkoholfraktionen gewonnen. Diese können allen in der Fachwelt für derartige Alkohole bekannten Verwendungszwecken zugeführt werden, insbesondere zur Synthese eines Polyesters. Gegebenenfalls vorhandene Amine können, wie für die erste Variante beschrieben weiterverarbeitet werden.

Enthält die dem Schritt (B) zugeführte Polymerzusammensetzung Anteile an nicht auf Polyester-Polyurethan-Elastomere zurückzuführende (insbesondere thermoplastische) Polyurethane, so können deren Abbauprodukte ebenfalls in die flüssige Alkoholphase gelangen. In diesem Fall ist die Durchführung einer Extraktion (erste Variante) bevorzugt. So können etwa Polyetherpolyole in die flüssige Alkoholphase gelangen. Bei der Extraktion gelangen Polyetherpolyole im Allgemeinen in die unpolare Phase. In diesem Fall sollte die optionale Destillation gemäß (E.I.d) durchgeführt werden, in welcher dann Polyetherpolyole als schwerer siedende Fraktion anfallen. Die so gewonnenen Polyetherpolyole können dann entweder stofflich wiederverwendet oder verbrannt werden, vorzugsweise unter energetischer Nutzung der freiwerdenden Verbrennungswärme. Wird auf die Extraktion verzichtet, fallen Polyetherpolyole analog in Schritt (E.II.b) an.

Enthält die Polymerzusammensetzung Polycarbonatbestandteile, so werden diese in Schritt (B) gespalten, insbesondere in Bisphenol-A und ein Carbonat des Chemolysealkohols. Beide Produkte gelangen bevorzugt in die flüssige Alkoholphase. Auch in diesem Fall ist die Aufarbeitung der flüssigen Alkoholphase gemäß der ersten Variante bevorzugt; ebenso ist die Durchführung von Schritt (E.I.d) bevorzugt. Bisphenol-A bzw. daraus entstandene Folgeprodukte wird/werden nach Durchlaufen der Hydrogenolyse in der Destillation (E.I.d) abgetrennt. Carbonate des Chemolysealkohols gehen bei der Extraktion bevorzugt in die polare Phase über und können, sofern sie sich nicht in den zugrundeliegenden Alkohol und CO₂ zersetzen, aus dieser durch Destillation abgetrennt und weiterverwendet werden, beispielsweise in der Herstellung neuer Polycarbonate.

In einer **dritten Variante** von Schritt (E) wird schließlich
(E.III) die in (C) erhaltene flüssige Alkoholphase (direkt) in der Synthese eines Polyesters eingesetzt.

Die Herstellung des Polyesters kann nach bekannten Methoden erfolgen. Zu der erhaltenen flüssigen Alkoholphase können weitere Säure- oder Alkoholkomponenten sowie Ester der Carbonsäuren mit niedermolekularen Alkoholen und Katalysatoren zugegeben werden, um die Stoffeigenschaften des Polyesters wie auch die Veresterungsreaktion selbst zu beeinflussen. Optional zugegeben werden können beispielsweise Carboxy-funktionelle Verbindungen und deren Derivate wie Adipinsäure, Bernsteinsäure, Therephthalsäure oder Sebacinsäure wie auch Verbindungen ausgewählt aus der Gruppe der Diole wie Monoethylenglykol, Diethylenglykol und Butandiol.

Als Katalysatoren kommen im Prinzip sämtliche für die Herstellung von Polyestern bekannte Katalysatoren in Frage. Dies sind beispielsweise Zinn-Salze, z.B. Zinndichlorid oder Zinndiethylhexanoat, Titanate, z. B. Tetrabutyltitanat oder Tyzor EZ oder starke Säuren, z.B. p-Toluolsulfonsäure, sowie Organozinnverbindungen, z. B. Dibutylzinndilaurat oder Salze des Bismuts, sowie die Carboxylate des Zinks, des Mangans, und anderer Übergangsmetalle. Polyesterpolyole können aber auch ohne den Einsatz von Katalysatoren hergestellt werden. Der Katalysator kann zu Beginn, oder im Laufe der Reaktion zugegeben werden und auch ggf. im Laufe der Reaktion gezielt desaktiviert werden. Bei der Veresterungsreaktion kann in an sich bekannter Weise so vorgegangen werden, dass mindestens die Edukte vorgelegt und durch Erhitzen zur Reaktion gebracht werden. Normalerweise werden die Polyesterpolyole ohne Verwendung eines zusätzlichen Lösungsmittels hergestellt. Das Austragen des Reaktionswassers sowie des freiwerdenden Chemolysealkohols wird bevorzugt durch Anlegen eines Unterdrucks, insbesondere gegen Ende der Veresterung, unterstützt. Das Austragen von Reaktionswasser und Chemolysealkohol kann auch durch Durchleiten eines inerten Gases, etwa Stickstoff oder Argon, unterstützt werden. Hierbei werden Drücke von 1 bis 500 mbar angewendet. Eine Veresterung ist jedoch auch oberhalb 500 mbar möglich. Die Veresterung kann jedoch auch unter Zusatz eines zusätzlichen Lösungsmittels erfolgen, insbesondere eines wasserschleppenden Lösungsmittel (Azeotropveresterung), wie etwa Benzol, Toluol oder Dioxan. Lösungsmittel können ebenfalls zugegeben werden, um den im niedermolekularen Ester enthaltenen Chemolysealkohol dem Reaktionsgemisch azeotrop zu entziehen.

Die Veresterung bzw. Umesterungsreaktion wird bevorzugt bei reduzierten Drücken von 1 mbar bis 600 mbar, insbesondere bei Drücken unter 500 mbar, durchgeführt. Die Reaktion kann in einer einstufigen oder mehrstufigen Veresterungsreaktion oder Umesterungsreaktion der Säurekomponenten und der alkoholischen Komponenten erfolgen.

### Beispiele:

Die in den Beispielen angegebenen Druckangaben sind relativ zum Atmosphärendruck angegeben ("Überdruck").

### Beispiel 1 (erfindungsgemäß; Chemolyse mit Methanol ohne Zusatz eines Chemolysekatalysators; Schritte (A) bis (C))

Ca. 500 g eines Polyester-Polyurethan-Elastomers (PEPUE) basierend auf einer Polyesterkomponente (A)(i) aus Adipinsäure, 1,4-Butandiol und Monoethylenglykol, einer Isocyanatkomponente (A)(ii) aus NDI, wobei die Isocyanatkomponente 20 Massen-% der Gesamtmasse des PEPUE ausmachte, und dem Kettenverlängerer (A)(iii) 1,4-Butandiol, wurden in einem Rührautoklaven mit 3 L Innenvolumen gegeben **(Schritt (A)).** Es wurden ca. 1000 g Methanol hinzugegeben, und der Reaktor wurde durch Begasen mit Stickstoff inertisiert. Anschließend wurde der Druck im Reaktor durch Stickstoffzugabe auf ca. 60 bar eingestellt. Die Drehzahl des Rührers wurde auf 400 U/min eingestellt, und der Reaktorinhalt mit einem Temperaturanstieg von 1°C/min auf 200 °C Innentemperatur aufgeheizt. Nach Erreichen dieser Temperatur wurde ein Druck von ca. 125 bar gemessen. Anschließend wurde der Reaktor bei gleicher Innentemperatur für 6 h gerührt. Dann wurde der Reaktorinhalt auf 25 °C abgekühlt, der Überdruck im Reaktor abgelassen und der Inhalt des Autoklaven bei Zimmertemperatur in eine Glasflasche überführt **(Schritt (B)).** Anschließend wurde der Feststoff abfiltriert, mit Methanol gewaschen und getrocknet **(Schritt (C)).**

Der Versuch wurde zweimal wiederholt und die jeweils erhaltenen festen Produkte wurden vereinigt. Insgesamt wurden so 549 g Feststoff erhalten, der zu 42 % (bestimmt per ¹HNMR) aus 1,5-NDI-Dimethylcarbamat bestand, entsprechend einer Ausbeute von 59 % der Theorie.

### Beispiel 2 (erfindungsgemäß; Chemolyse mit Ethanol ohne Zusatz eines Chemolysekatalysators; Schritte (A) bis (C))

72,3 g des gleichen PEPUE wie in Beispiel 1 wurden in analoger Vorgehensweise mit 197,6 g Ethanol umgesetzt. Es wurden 30,4 g eines klebrigen Feststoffes erhalten, der (bestimmt per ¹HNMR) 20,3 g 1,5-NDI-Dimethylcarbamat enthielt.

### Beispiel 3 (erfindungsgemäß, basische Hydrolyse von NDI-Dimethylcarbamat; Schritt (D.I))

255,1 g des Feststoffs aus Beispiel 1 wurden mit 94,5 g Natriumhydroxid-Pellets und 2070 g Wasser (vollentsalzt, "VE") in einen druckbeständigen Rührautoklaven (Innenvolumen 3 L) gegeben. Der Reaktor wurde mit Stickstoff inertisiert und vor dem Aufheizen auf eine Druck von 5 bar eingestellt. Nun wurde der Reaktorinhalt mit einer Heizrate von ca. 1 °C/min auf die gewünschte Reaktionstemperatur von 220 °C erhitzt. Nach Überschreiten von 100 °C wurde der Rührer mit einer Drehzahl von 400 U/min zugeschaltet. Nach Erreichen der Reaktionstemperatur von 220 °C wurde diese für 1 h gehalten. Hierbei stellte sich ein max. Druck von ca. 28 bar ein. Anschließend wurde der Reaktorinhalt innerhalb von 2 h auf Raumtemperatur abgekühlt und anschließend auf Umgebungsdruck entspannt. Nach dem Öffnen des Reaktors wurde die gesamte Reaktionsmischung mit Stickstoff überschleiert und mit Hilfe einer Pumpe in eine Glasflasche überführt. Anschließend wurde über eine Fritte der erhaltene braune Feststoff abfiltriert, mit Wasser pH-neutral gewaschen und in einem Vakuumtrockenschrank bei 50°C und 100 mbar über Nacht getrocknet. Auf diese Weise wurden 59 g Feststoff isoliert. Analyse dieses Feststoffes mittels Gaschromatographie ergab, dass dieser zu 99,5 % aus 1,5 -NDA bestand, was einer Ausbeute von ca. 96 % der Theorie (bezogen auf das im in der Hydrolyse eingesetzten Feststoff aus Beispiel 1 enthaltene 1,5-NDI-Dimethylcarbamat) entspricht.

### Beispiel 4 (Extraktion von Adipinsäuredimethylester (ADM); Schritt (E.I.a))

Ein Teil der in Beispiel 1 bei der Abtrennung des Feststoffs verbleibenden flüssigen Alkoholphase wurde durch Destillation von Methanol befreit. Dabei verblieb eine Mischung mit einem hohen Anteil an Adipinsäuredimethylester (67 Massen-%) sowie Monoethylenglykol und 1,4-Butandiol. Die Mischung, die bei Raumtemperatur zweiphasig war, wurde mit verschiedenen Extraktionsmitteln im Massen-Verhältnis 1 : 1 einstufig extrahiert (Beispiele 4a bis 4c). In zwei Versuchen (Beispiele 4d und 4e) wurde ein Extraktionsmittel aus einem organischen Lösungsmittel und Wasser eingesetzt; in diesen Fällen wurden die Mischung, das organische Lösungsmittel und das Wasser im Verhältnis 1 : 1 : 1 eingesetzt. Die Extraktion wurde bei 20 °C durchgeführt. Zur Bewertung der Versuche wurden die **Verteilungskoeffizienten *K*** (= Verhältnis des ADM-Massenanteils in der ADM-reichen Phase zum ADM-Massenanteil in der ADM-armen Phase) bestimmt. Die Verteilungen von Monoethylenglykol (MEG) und 1,4-Butandiol (BDO) wurden ebenfalls bestimmt.

**Tabelle 1: Verteilungskoeffizienten von ADM, MEG und BDO**

| **Bsp.** | **Extraktionsmittel** | ***K_{ADM} = w(ADM)ᵤₙₚₒₗₐᵣ* / *w(ADM)ₚₒₗₐᵣ*** | ***K = w(MEG)ᵤₙₚₒₗₐᵣ* / *w(MEG)ₚₒₗₐᵣ*** | ***K= w(BDO)ᵤₙₚₒₗₐᵣ*** / ***w(BDO)ₚₒₗₐᵣ*** |
|---|---|---|---|---|
| 4a | Monochlorbenzol | 10,784 | 0,019 | 0,022 |
| 4b | Hexan | 3,75 | 0,005 | 0,007 |
| 4c | Wasser | 35,286 | 0,023 | 0,041 |
| 4d | Monochlorbenzol und Wasser | 50,625 | 0,006 | < 0,005 |
| 4e | Hexan und Wasser | 13,333 | < 0,005 | < 0,005 |

| | | | | |
|---|---|---|---|---|
| Erläuterungen: w = Massenanteil; V = Vergleichsbeispiel. | | | | |

Die Extraktion von ADM aus der von Methanol befreiten flüssigen Alkoholphase gelingt mit Monochlorbenzol (Beispiel 4a), wobei MEG und BDO weitgehend zurückbleiben und die Hauptbestandteile der polaren Phase bilden. Ein unpolares Extraktionsmittel wie Hexan führt zu weniger guten Ergebnissen (siehe Beispiel 4b; K_{ADM} ist kleiner als in Beispiel 4a), ist aber trotzdem noch einsetzbar. Der Einsatz von Wasser in Kombination mit einem wenig polaren (Monochlorbenzol) bzw. unpolaren (Hexan) organischen Lösungsmittel (Beispiele 4d und 4e) verbessert die Ergebnisse im Vergleich zum Einsatz dieser Lösungsmittel alleine (Beispiel 4a und 4b).

Wasser alleine eignet sich ebenfalls als Extraktionsmittel (Beispiel 4c), wobei in diesem Fall nicht ADM, sondern MEG und BDO aus der von Methanol befreiten flüssigen Alkoholphase herausextrahiert werden. Die Anteile von MEG und BDO in der ADM-Phase sind im Vergleich zu den anderen Beispielen leicht erhöht.

Bei geeigneter Prozessführung (z. B. durch mehrstufige Extraktion) können alle diese Werte noch optimiert werden.

### Beispiel 5 (erfindungsgemäß, Chemolyse mit Methanol ohne Zusatz eines Chemolysekatalysators, gefolgt von Hydrogenolyse des gebildeten Adipinsäuredimethylesters; Schritte (A) bis (C) und (E.II.a))

Ca. 167 g eines Polyester-Polyurethan-Elastomers (PEPUE) basierend auf einer Polyesterkomponente (A)(i) aus Adipinsäure, 1,4-Butandiol und Monoethylenglykol, einer Isocyanatkomponente (A)(ii) aus NDI, wobei die Isocyanatkomponente 20 Massen-% der Gesamtmasse des PEPUE ausmachte, und dem Kettenverlängerer (A)(iii) 1,4-Butandiol, wurden in einem Rührautoklaven mit 1 L Innenvolumen gegeben **(Schritt (A)).** Es wurden ca. 333 g Methanol hinzugegeben, und der Reaktor wurde durch Begasen mit Stickstoff inertisiert. Das Reaktionsgemisch wurde unter Rühren (500 U/min) auf eine Reaktionstemperatur von 200 °C erhitzt, wobei der Druck auf bis zu 40 bar stieg. Nach 180 min Reaktionszeit bei 200 °C wurde der Reaktor abgekühlt, und nach Erreichen einer Innentemperatur von ca. 25 °C entspannt. Das Produkt wurde anschließend ausgetragen **(Schritt (B)).** Der entstandene Feststoff wurde durch Filtration abgetrennt, mit MeOH gewaschen und anschließend unter Vakuum getrocknet **(Schritt (C)).**

Ein Teil der bei der Abtrennung des Feststoffs verbleibenden flüssigen Alkoholphase wurde in Gegenwart eines heterogenen Kupferoxidkatalysators (Ranido RCAT-2200, 20 Massen-% bezogen auf die Menge an Adipinsäuredimethylester) einer Hydrogenolyse unterzogen **(Schritt (E.II.a)).** Diese Flüssigphase war wie folgt zusammengesetzt: 70,9 Massen-% MeOH, 6,2 Massen-% Monoethylenglykol, 2,3 Massen-% 1,4-Butandiol, 18,6 Massen-% Adipinsäuredimethylester und in Summe 2,0 Massen-% Mono- und Bis-NDI-Methylcarbamat.

2,00 ml der alkoholischen Flüssigphase wurden mit 20 mg n-Tetradecan (GC-Standard) in einem 20-ml-Hochdruckautoklaven vorgelegt. Nach Inertisierung des Reaktors mit Wasserstoff wurde der Druck mit Wasserstoff auf 70 bar eingestellt, und die Reaktionsmischung wurde bei einer Temperatur von 220 °C in einem Aluminium-Heizblock für 16 h gerührt (900 U/min). Anschließend wurde der Reaktor in einem Eisbad gekühlt und belüftet. Die Reaktionsmischung wurde mit 1,4-Dioxan (2 ml) verdünnt und filtriert. Nach Filtration wurde eine Probe der Reaktionsmischung entnommen und mittels Gaschromatographie analysiert. Es wurde eine Ausbeute an 1-Hexansäuremethylester-6-ol von 45,4 % und 1,6-Hexandiol von 51,9 % bestimmt.

## Patentansprüche

1. Verfahren zur Wiedergewinnung von Rohstoffen aus Polyester-Polyurethan-Elastomer-haltigen Polymerzusammensetzungen, umfassend die Schritte:
(A) Bereitstellen einer Polymerzusammensetzung enthaltend ein
auf (i) einer Polyesterkomponente, (ii) einer Isocyanatkomponente und optional (iii) einem Kettenverlängerer und/oder Vernetzer mit Hydroxy-und/oder Aminfunktionalität basierendes
Polyester-Polyurethan-Elastomer,
wobei die Polymerzusammensetzung ein erstes Polyester-Polyurethan-Elastomer enthält, dessen Isocyanatkomponente Naphthylen-1,5-diisocyanat umfasst;
(B) Umsetzen der Polymerzusammensetzung mit einem linearen primären Mono-Alkohol mit 1 bis 4 Kohlenstoffatomen unter Erhalt eines Produktgemisches umfassend
eine flüssige Alkoholphase;
und
eine feste Carbamatphase;
und
(C) Isolieren des Carbamats aus dem Produktgemisch.

2. Verfahren nach Anspruch 1, bei welchem die Polymerzusammensetzung neben dem ersten Polyester-Polyurethan-Elastomer kein weiteres Polyester-Polyurethan-Elastomer enthält.

3. Verfahren nach Anspruch 1, bei welchem die Polymerzusammensetzung ein zweites Polyester-Polyurethan-Elastomer enthält, dessen Isocyanatkomponente kein Naphthylen-1,5-diisocyanat, aber Methylendiphenylendiisocyanat und/oder Toluylendiisocyanat umfasst.

4. Verfahren nach Anspruch 3, bei welchem der Massenanteil des ersten Polyester-Polyurethan-Elastomers, bezogen auf die Gesamtmasse des ersten und zweiten Polyester-Polyurethan-Elastomers, im Bereich von 60 % bis < 100 % liegt.

5. Verfahren nach Anspruch 3 oder 4, bei welchem die Polymerzusammensetzung neben dem ersten und zweiten Polyester-Polyurethan-Elastomer kein weiteres Polyester-Polyurethan-Elastomer enthält.

6. Verfahren nach einem der vorstehenden Ansprüche, bei welchem der Massenanteil an Polyester-Polyurethan-Elastomer in der Polymerzusammensetzung im Bereich von 60 % bis 100 % liegt.

7. Verfahren nach Anspruch 6, bei welchem ein nicht auf Polyester-Polyurethan-Elastomer zurückzuführender Anteil der Polymerzusammensetzung Polyurethane umfasst, die auf einer Polyetherpolyol-umfassenden und/oder Polycarbonatpolyol-umfassenden Polyolkomponente und einer Methylendiphenylendiisocyanat-umfassenden und/oder Toluylendiisocyanat-umfassenden Isocyanatkomponente basieren.

8. Verfahren nach einem der vorstehenden Ansprüche, umfassend:
(D) Weitere Umsetzung des in (C) isolierten Carbamats, wobei die weitere Umsetzung eine der folgenden umfasst:
(D.I) Hydrolyse des Carbamats mit Wasser in Gegenwart eines Hydrolyse-Katalysators unter Bildung von Naphthylen-1,5-diamin und des linearen primären Mono-Alkohols;
(D.II) Spalten des Carbamats in Naphthylen-1,5-diisocyanat und den linearen primären Mono-Alkohol;
(D.III) Hydrogenolyse des Carbamats mit Wasserstoff in Gegenwart eines Hydrogenolyse-Katalysators unter Bildung von Naphthylen-1,5-diamin und des linearen primären Mono-Alkohols;
oder
(D.IV) Umsetzung des Carbamats mit einem Polyol in Gegenwart oder Abwesenheit eines Katalysators zu einem OH-terminierten Präpolymer.

9. Verfahren nach Anspruch 8, umfassend Schritt (D.I) oder (D.III), bei welchem das Naphthylen-1,5-diamin, gegebenenfalls nach einer Reinigung, mit Phosgen zu Naphthylen-1,5-diisocyanat umgesetzt wird.

10. Verfahren nach einem der vorstehenden Ansprüche, bei welchem Schritt (C) umfasst:
Trennung der festen Carbamatphase von der flüssigen Alkoholphase, optional gefolgt von einer Wäsche sowie erforderlichenfalls von weiteren Reinigungsschritten der festen Carbamatphase, zur Isolierung des Carbamats.

11. Verfahren nach Anspruch 10, umfassend eine Aufarbeitung der flüssigen Alkoholphase gemäß einer der folgenden Varianten (E.I), (E.II) oder (E.III),
wobei (E.I) umfasst:
(E.I.a) Extraktion der flüssigen Alkoholphase, optional nach destillativer Abtrennung von darin enthaltenem linearen primären Mono-Alkohol, mit einem organischen und/oder wässrigen Extraktionsmittel und Trennung in eine unpolare Phase und eine polare Phase;
(E.I.b) Optional, Verdampfung von gegebenenfalls vorhandenem Extraktionsmittel und/oder gegebenenfalls vorhandenem linearen primären Mono-Alkohol aus der unpolaren Phase unter Verbleib einer aufkonzentrierten organischen Phase;
(E.I.c) Umsetzung der unpolaren Phase oder der aufkonzentrierten organischen Phase mit Wasserstoff in Gegenwart eines Hydrogenolyse-Katalysators unter Erhalt eines Alkohols;
und optional
(E.I.d) weitere Aufreinigung des Alkohols durch Destillation;
wobei (E.II) umfasst:
(E.II.a) Umsetzung der flüssigen Alkoholphase mit Wasserstoff in Gegenwart eines Hydrogenolyse-Katalysators, wobei der Umsetzung mit Wasserstoff eine destillative Abtrennung von linearen primären Mono-Alkohol vorangeht und/oder folgt, sodass ein Gemisch aus Alkoholen verbleibt;
und
(E.II.b) destillative Trennung des Gemisches aus Alkoholen in einzelne Alkohol-Fraktionen;
und wobei (E.III) umfasst:
Einsetzen der flüssigen Alkoholphase in der Synthese eines Polyesters.

12. Verfahren nach Anspruch 11, umfassend die Variante (E.I), wobei der Alkohol aus (E.I.c) oder (E.I.d) in der Synthese eines Polyesters eingesetzt wird.

13. Verfahren nach Anspruch 11, umfassend die Variante (E.I), oder nach Anspruch 12, wobei die polare Phase zur Gewinnung einer Alkoholkomponente der Polyesterkomponente und/oder von optional vorhandenem Kettenverlängerer und/oder Vernetzer aufgearbeitet wird.

14. Verfahren nach Anspruch 13, bei welchem die aus der polaren Phase gewonnene Alkoholkomponente und/oder der aus der polaren Phase gewonnene Kettenverlängerer und/oder Vernetzer in der Herstellung neuen Polyester-Polyurethan-Elastomers eingesetzt werden/wird.

15. Verfahren nach Anspruch 11, umfassend Variante (E.II), wobei eine oder mehrere der Alkohol-Fraktionen aus (E.II.b) in der Synthese eines Polyesters eingesetzt werden.

## Claims

1. Process for recovering raw materials from polyester-polyurethane elastomer-containing polymer compositions, comprising the steps of:
(A) providing a polymer composition containing a polyester-polyurethane elastomer
based on (i) a polyester component, (ii) an isocyanate component and optionally (iii) a chain extender and/or crosslinker having hydroxy and/or amine functionality,
where the polymer composition contains a first polyester-polyurethane elastomer wherein the isocyanate component comprises naphthylene 1,5-diisocyanate;
(B) reacting the polymer composition with a linear primary monoalcohol having 1 to 4 carbon atoms to obtain a product mixture comprising
a liquid alcohol phase;
and
a solid carbamate phase;
and
(C) isolating the carbamate from the product mixture.

2. Process according to Claim 1, in which the polymer composition does not contain any polyester-polyurethane elastomer aside from the first polyester-polyurethane elastomer.

3. Process according to Claim 1, in which the polymer composition contains a second polyester-polyurethane elastomer wherein the isocyanate component comprises not naphthylene 1,5-diisocyanate but methylene diphenylene diisocyanate and/or tolylene diisocyanate.

4. Process according to Claim 3, in which the proportion by mass of the first polyester-polyurethane elastomer, based on the total mass of the first and second polyester-polyurethane elastomers, is in the range from 60% to < 100%.

5. Process according to Claim 3 or 4, in which the polymer composition does not contain any polyester-polyurethane elastomer aside from the first and second polyester-polyurethane elastomers.

6. Process according to any of the preceding claims, in which the proportion by mass of polyester-polyurethane elastomer in the polymer composition is in the range from 60% to 100%.

7. Process according to Claim 6, in which any proportion of the polymer composition not originating from polyester-polyurethane elastomer comprises polyurethanes based on a polyether polyol-comprising and/or polycarbonate polyol-comprising polyol component and a methylene diphenylene diisocyanate-comprising and/or tolylene diisocyanate-comprising isocyanate component.

8. Process according to any of the preceding claims, comprising:
(D) further converting the carbamate isolated in (C), wherein the further conversion comprises one of the following:
(D.I) hydrolysing the carbamate with water in the presence of a hydrolysis catalyst to form naphthylene-1,5-diamine and the linear primary monoalcohol;
(D.II) cleaving the carbamate to naphthylene 1,5-diisocyanate and the linear primary monoalcohol;
(D.III) hydrogenolysing the carbamate with hydrogen in the presence of a hydrogenolysis catalyst to form naphthylene-1,5-diamine and the linear primary monoalcohol;
or
(D.IV) reacting the carbamate with a polyol in the presence or absence of a catalyst to give an OH-terminated prepolymer.

9. Process according to Claim 8, comprising step (D.I) or (D.III), in which the naphthylene-1,5-diamine, optionally after purification, is reacted with phosgene to give naphthylene 1,5-diisocyanate.

10. Process according to any of the preceding claims, in which step (C) comprises:
separating the solid carbamate phase from the liquid alcohol phase, optionally followed by a wash and, if required, by further purification steps on the solid carbamate phase to isolate the carbamate.

11. Process according to Claim 10, comprising a workup of the liquid alcohol phase according to one of the following variants (E.I), (E.II) and (E.III),
where (E.I) comprises:
(E.I.a) extracting the liquid alcohol phase, optionally after distillative removal of linear primary monoalcohol present therein, with an organic and/or aqueous extractant, and separating into a nonpolar phase and a polar phase;
(E.I.b) optionally evaporating any extractant present and/or any linear primary monoalcohol present out of the nonpolar phase to leave a concentrated organic phase;
(E.I.c) reacting the nonpolar phase or the concentrated organic phase with hydrogen in the presence of a hydrogenolysis catalyst to obtain an alcohol;
and optionally
(E.I.d) further purifying the alcohol by distillation;
where (E.II) comprises:
(E.II.a) reacting the liquid alcohol phase with hydrogen in the presence of a hydrogenolysis catalyst, where the reaction with hydrogen is preceded and/or followed by a distillative removal of linear primary monoalcohol, so as to leave a mixture of alcohols;
and
(E.II.b) distillatively separating the mixture of alcohols into individual alcohol fractions;
and where (E.III) comprises:
using the liquid alcohol phase in the synthesis of a polyester.

12. Process according to Claim 11, comprising variant (E.I), where the alcohol from (E.I.c) or (E.I.d) is used in the synthesis of a polyester.

13. Process according to Claim 11, comprising variant (E.I), or according to Claim 12, wherein the polar phase is worked up to obtain an alcohol component of the polyester component and/or optionally present chain extender and/or crosslinker.

14. Process according to Claim 13, in which the alcohol component obtained from the polar phase and/or the chain extender and/or crosslinker obtained from the polar phase is/are used in the production of new polyester-polyurethane elastomer.

15. Process according to Claim 11, comprising variant (E.II), where one or more of the alcohol fractions from (E.II.b) are used in the synthesis of a polyester.

## Revendications

1. Procédé de récupération de matières premières à partir de compositions polymères contenant un élastomère de polyester-polyuréthane, comprenant les étapes suivantes :
(A) la fourniture d'une composition polymère contenant un élastomère de polyester-polyuréthane à base de
(i) un composant polyester, (ii) un composant isocyanate et, éventuellement, (iii) un allongeur de chaîne et/ou un agent de réticulation présentant une fonctionnalité hydroxy et/ou amine,
la composition polymère contenant un premier élastomère de polyester-polyuréthane dont le composant isocyanate comprend du diisocyanate de naphtylène-1,5 ;
(B) la mise en réaction de la composition polymère avec un monoalcool primaire linéaire comportant de 1 à 4 atomes de carbone, de manière à obtenir un mélange de produits comprenant
une phase alcoolique liquide ;
et
une phase de carbamate solide ;
et
(C) l'isolement du carbamate du mélange de produits.

2. Procédé selon la revendication 1, dans lequel la composition polymère ne contient, outre le premier élastomère de polyester-polyuréthane, aucun autre élastomère de polyester-polyuréthane.

3. Procédé selon la revendication 1, dans lequel la composition polymère contient un deuxième élastomère de polyester-polyuréthane dont le composant isocyanate ne comprend pas de diisocyanate de naphtylène-1,5, mais comprend du diisocyanate de diphénylméthane et/ou du diisocyanate de toluylène.

4. Procédé selon la revendication 3, dans lequel la fraction massique du premier élastomère de polyester-polyuréthane, rapportée à la masse totale du premier et du deuxième élastomère de polyester-polyuréthane, est dans la plage allant de 60 % à < 100 %.

5. Procédé selon la revendication 3 ou 4, dans lequel la composition polymère ne contient, outre le premier et le deuxième élastomère de polyester-polyuréthane, aucun autre élastomère de polyester-polyuréthane.

6. Procédé selon l'une des revendications précédentes, dans lequel la fraction massique d'élastomère de polyester-polyuréthane dans la composition polymère est dans une plage allant de 60 % à 100 %.

7. Procédé selon la revendication 6, dans lequel une fraction de la composition polymère ne provenant pas de l'élastomère de polyester-polyuréthane comprend des polyuréthanes à base d'un composant polyol comprenant un polyétherpolyol et/ou un polycarbonatepolyol et d'un composant isocyanate comprenant du diisocyanate de diphénylméthane et/ou du diisocyanate de toluylène.

8. Procédé selon l'une des revendications précédentes, comprenant :
(D) la mise en réaction supplémentaire du carbamate isolé en (C), la mise en réaction supplémentaire comprenant l'une des opérations suivantes :
(D.I) l'hydrolyse du carbamate avec de l'eau en présence d'un catalyseur d'hydrolyse, avec formation de naphtylène-1,5-diamine et du monoalcool primaire linéaire ;
(D.II) le clivage du carbamate en diisocyanate de naphtylène-1,5 et le monoalcool primaire linéaire ;
(D.III) l'hydrogénolyse du carbamate avec de l'hydrogène en présence d'un catalyseur d'hydrogénolyse, avec formation de naphtylène-1,5-diamine et du monoalcool primaire linéaire ;
ou
(D.IV) la mise en réaction du carbamate avec un polyol en présence ou en l'absence d'un catalyseur, en vue d'obtenir un prépolymère à terminaison OH.

9. Procédé selon la revendication 8, comprenant l'étape (D.I) ou (D.III), dans lequel la naphtylène-1,5-diamine est, le cas échéant après purification, mise en réaction avec du phosgène pour former du diisocyanate de naphtylène-1,5.

10. Procédé selon l'une des revendications précédentes, dans lequel l'étape (C) comprend :
la séparation de la phase de carbamate solide de la phase alcoolique liquide, éventuellement suivie d'un lavage ainsi que, si nécessaire, d'autres étapes de purification de la phase de carbamate solide, afin d'isoler le carbamate.

11. Procédé selon la revendication 10, comprenant un traitement de la phase alcoolique liquide selon l'une des variantes (E.I), (E.II) ou (E.III) suivantes,
(E.I) comprenant :
(E.I.a) l'extraction de la phase alcoolique liquide, éventuellement après séparation par distillation du monoalcool primaire linéaire qu'elle contient, au moyen d'un agent d'extraction organique et/ou aqueux, et la séparation en une phase apolaire et une phase polaire ;
(E.I.b) éventuellement, l'évaporation de l'agent d'extraction éventuellement présent et/ou du monoalcool primaire linéaire éventuellement présent de la phase apolaire, de manière à laisser une phase organique concentrée ;
(E.I.c) la mise en réaction de la phase apolaire ou de la phase organique concentrée avec de l'hydrogène en présence d'un catalyseur d'hydrogénolyse, de manière à obtenir un alcool ; et éventuellement
(E.I.d) la purification supplémentaire de l'alcool par distillation ;
(E.II) comprenant :
(E.II.a) la mise en réaction de la phase alcoolique liquide avec de l'hydrogène en présence d'un catalyseur d'hydrogénolyse, la réaction avec l'hydrogène étant précédée et/ou suivie d'une séparation par distillation du monoalcool primaire linéaire, de sorte qu'il reste un mélange d'alcools ;
et
(E.II.b) la séparation par distillation du mélange d'alcools en fractions d'alcools individuelles ;
et (E.III) comprenant :
l'utilisation de la phase alcoolique liquide dans la synthèse d'un polyester.

12. Procédé selon la revendication 11, comprenant la variante (E.I), dans lequel l'alcool obtenu à l'étape (E.I.c) ou (E.I.d) est utilisé dans la synthèse d'un polyester.

13. Procédé selon la revendication 11, comprenant la variante (E.I), ou selon la revendication 12, dans lequel la phase polaire est traitée en vue de récupérer un composant alcool de la composante polyester et/ou l'allongeur de chaîne et/ou l'agent de réticulation éventuellement présent.

14. Procédé selon la revendication 13, dans lequel le composant alcoolique récupéré à partir de la phase polaire et/ou l'allongeur de chaîne et/ou l'agent de réticulation récupéré à partir de la phase polaire est/sont utilisé(s) pour la fabrication d'un nouvel élastomère de polyester-polyuréthane.

15. Procédé selon la revendication 11, comprenant la variante (E.II), dans lequel une ou plusieurs des fractions d'alcools issues de (E.II.b) sont utilisées dans la synthèse d'un polyester.
